# EUROPEAN PATENT SPECIFICATION

(11) **EP 2 315 849 B1**
(45) Date of publication and mention of the grant of the patent: **08.11.2017**
(21) Application number: 09803662.7
(22) Date of filing: 31.07.2009
(51) Int. Cl.: C12Q 1/68

(54) **CIRCULATING MUTANT DNA TO ASSESS TUMOR DYNAMICS**
ZIRKULIERENDE MUTANTEN-DNA ZUR BEWERTUNG VON TUMORDYNAMIKEN
ADN MUTANT CIRCULANT POUR ÉVALUER UNE DYNAMIQUE DE TUMEUR

(30) Priority: 31.07.2008 US 85175 P; 30.07.2009 US 512585
(43) Date of publication of application: 04.05.2011
(73) Proprietor: The Johns Hopkins University, Baltimore, MD 21201 (US)
(72) Inventor: DIEHL, Frank, 26419 Schortens (DE); DIAZ, Luis, Elkridge, MD 21075 (US); KINZLER, Kenneth, W., Baltimore, MD 21015 (US); VOGELSTEIN, Bert, Baltimore, MD 21208 (US); SCHMIDT, Kerstin, Nashville, TN 37212 (US)
(74) Representative: Tombling, Adrian George
(86) International application number: PCT/US2009/052436
(87) International publication number: WO 2010/014920

(56) References cited:
- US-A1- 2003 143 600
- DIEHL F ET AL.: "Circulating mutant DNA to assess tumor dynamics", NATURE MEDICINE, vol. 14, no. 9, 31 July 2008 (2008-07-31), pages 985-990, XP002666722,
- DIEHL FRANK ET AL: "Detection and quantification of mutations in the plasma of patients with colorectal tumors", PROCEEDINGS OF THE NATIONAL ACADEMY OF SCIENCES OF USA, NATIONAL ACADEMY OF SCIENCE, WASHINGTON, DC; US, [Online] vol. 102, no. 45, 8 November 2005 (2005-11-08), pages 16368-16373, XP002518285, ISSN: 0027-8424, DOI: 10.1073/PNAS.0507904102 Retrieved from the Internet: URL:10.1073/pnas.0507904102>
- RYAN B M ET AL.: "A prospective study of circulating mutant KRAS2 in the serum of patients with colorectal neoplasia: string prognostic indicator in postoperative follow up", GUT, vol. 52, no. 1, January 2003 (2003-01), pages 101-108, XP002666723,
- MENG LI ET AL: "BEAMing up for detection and quantification of rare sequence variants", NATURE METHODS, NATURE PUBLISHING GROUP, GB, vol. 3, no. 2, 1 February 2006 (2006-02-01), pages 95-97, XP008126872, ISSN: 1548-7091, DOI: 10.1038/NMETH850
- HESS SCHMID M ET AL.: "Tumor burden index as a prognostic tool for cutaneous T-cell lymphoma", ARCHIVES IN DERMATOLOGY, vol. 135, 1999, pages 1204-1208, XP002666724,
- DIEHL ET AL.: 'Analysis of Mutations in DNA Isolated From Plasma and Stool of Colorectal Cancer Patients.' GASTROENTEROLOGY. vol. 135, no. 2, 15 May 2008, pages 489 - 498, XP023901355
- C. Rago ET AL: "Serial Assessment of Human Tumor Burdens in Mice by the Analysis of Circulating DNA", Cancer Research, vol. 67, no. 19, 1 October 2007 (2007-10-01), pages 9364-9370, XP055288572, US ISSN: 0008-5472, DOI: 10.1158/0008-5472.CAN-07-0605

## Description

### TECHNICAL FIELD OF THE INVENTION

This invention is related to the area of cancer. In particular, it relates to cancer diagnosis, prognosis, therapeutics, and monitoring.

### BACKGROUND OF THE INVENTION

Cancers arise through the sequential alteration of genes that control cell growth. In solid tumors such as those of the colon or breast, it has been shown that, on average, approximately 80 genes harbor subtle mutations that are present in virtually every tumor cell but are not present in normal cells¹. These somatic mutations thereby have the potential to serve as highly specific biomarkers. They are, in theory, much more specific indicators of neoplasia than any other biomarker yet described. One challenge for modem cancer research is therefore to exploit somatic mutations as tools to improve the detection of disease and, ultimately, to positively affect individual outcomes.

Tumor cells can often be found in the circulation of individuals with advanced cancers ^{2,3}. It has been shown that tumor-derived mutant DNA can also be detected in the cell-free fraction of the blood of people with cancer⁴⁻⁶. Most of this mutant DNA is not derived from circulating tumor cells⁴⁻⁶ and, in light of the specificity of mutations, raises the possibility that the circulating mutant DNA fragments themselves can be used to track disease. However, the reliable detection of such mutant DNA fragments is challenging⁷.

In particular, the circulating mutant DNA represents only a tiny fraction of the total circulating DNA, sometimes less than 0.01%^{g}.

In the current study, we developed modifications of a technique called BEAMing (Beads, Emulsion, Amplification and Magnetics)^{8,9} to quantify ctDNA in serially collected plasma samples from subjects with colorectal cancers. We were interested in determining whether such measurements provided information about the dynamics of tumor burden in these subjects during the course of their disease.

Diel et al. (2005) PNAS 102 (45), 16368 - 16373 and Diel et al. (2008) Gastroenterology 135 (2), 489-498 both disclose BEAMing generally and the quantification of DNA fragments. However, neither document discloses that relative tumor dynamics can be monitored by the use of circulating mutant DNA in blood or stool. C. Rago et al. (2007) Cancer Research, vol. 67, no. 19, pages 9364-9370 discloses that circulating hLINE-1 levels correlate with tumor burden in mice with human tumor xenografts. However, this document does not disclose the use of a somatic mutation in a gene selected from the group consisting of APC, KRAS, TP53, or PIKSCA for monitoring relative tumor dynamics.

There is a continuing need in the art for ways to better determine which patients will experience relapses of their cancer and which will not.

### SUMMARY OF THE INVENTION

According to one embodiment of the invention, a method is provided to monitor tumor dynamics in accordance with claim 1.

This and other embodiments which will be apparent to those of skill in the art upon reading the specification provide the art with methods which are useful for cancer patient management and monitoring.

### BRIEF DESCRIPTION OF THE DRAWINGS

Figs. 1A-1C show measurement of ctDNA. Fig. IB depicts conventional Sanger sequencing of DNA derived from the subject's tumor, representing the first step of the analysis. The approach for quantifying tumor-derived DNA in plasma samples is shown in Fig. 1C. Real-time PCR is used to measure the total number of DNA fragments in the plasma, whereas BEAMing measures the ratio of mutant to wild-type fragments labeled with Cy5 and Cy3 are fluorescent probes.

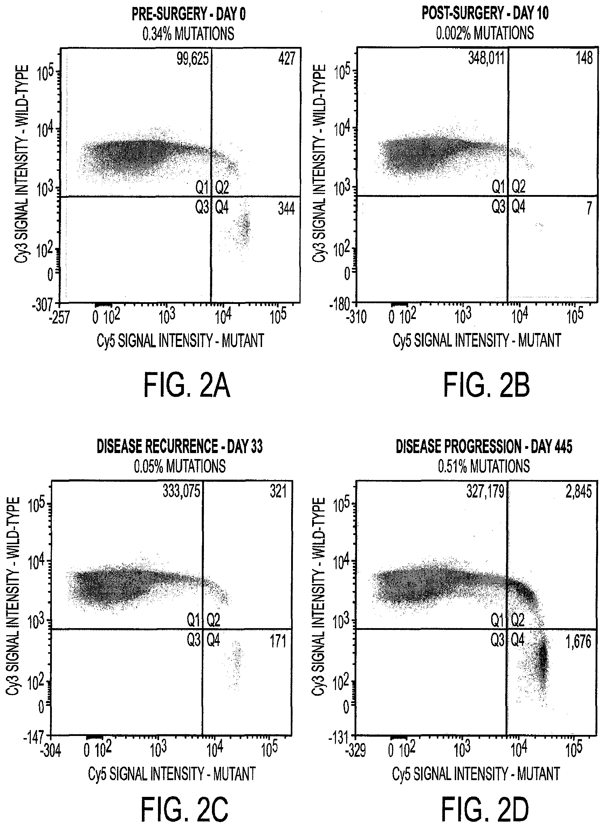

Fig. 2A to 2D shows representative flow cytometric data obtained from BEAMing. The four graphs illustrate the data obtained from subject 6 (*APC* G4189T) at different time points during treatment. The northwest quandrant dots and southeast quandrant dots represent beads bound to wild-type and mutant fragments, respectively. The northeast quandrant dots represent beads bound to both wild-type and mutant fragments resulting from their inclusion in an emulsion microdroplet that contained both wild-type and mutant DNA templates¹⁵. Numbers in each quadrant represent absolute counts of beads for each population measured. (Fig. 2A) Before surgery, the fraction of mutant DNA fragments was 13.4%. (Fig. 2B) After surgery (day 3), the fraction of mutant DNA fragments dropped to 0.015%. (Fig. 2C) After surgery (day 48), the fraction of mutant DNA fragments increased to 0.11%, suggesting disease recurrence. (Fig. 2D) On day 244, the subject had progressive disease and the fraction mutant DNA fragments increased further to 0.66%.
Figs. 3A-3B show recurrence-free survival, as detected by ctDNA and CEA. (Fig. 3A) The difference in recurrence-free survival in subjects with detectable versus undetectable post-operative ctDNA levels (P = 0.006 by Mantel-Cox log-rank test). (Fig. 3B) The difference in recurrence-free survival in subjects with detectable versus undetectable post-operative CEA levels (P = 0.03 by Mantel-Cox log-rank test).

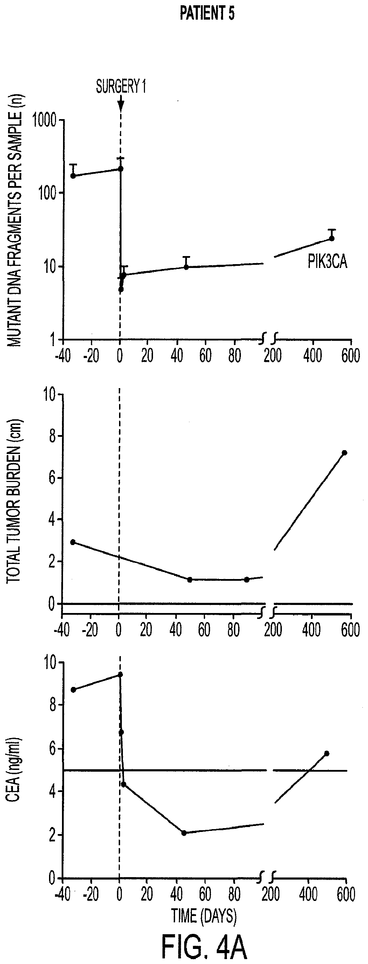

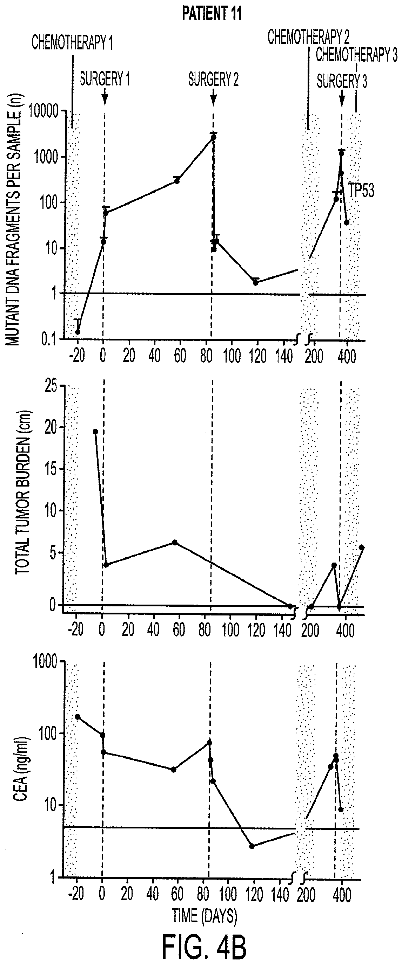

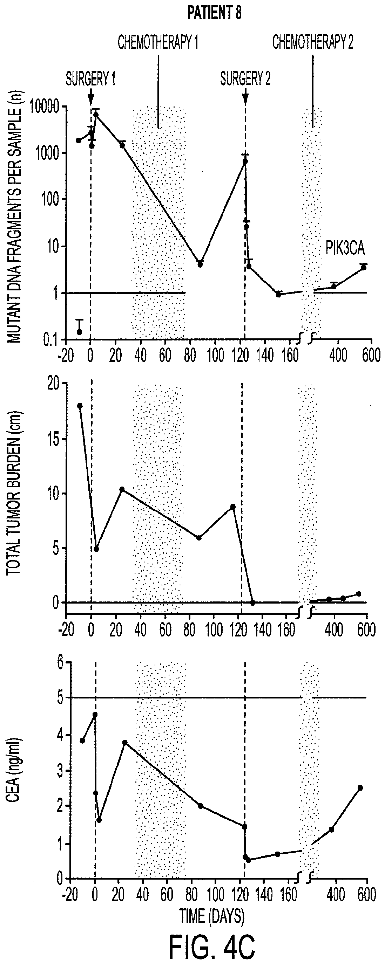

Figs. 4A-4C show comparison of ctDNA, CEA and imaging dynamics in individual study subjects. For each subject, the top, middle, and bottom graphs represent ctDNA level, tumor volume as assessed by imaging, and CEA level. The horizontal lines represent the upper bound of the normal levels: one mutant DNA fragment per sample for ctDNA levels, 0.0 cm for tumor diameter, and 5.0 ng ml⁻¹ for CEA abundance. Fig. 4C: Patient 8 had a sigmoid adenocarcinoma and solitary metastases in both hepatic lobes. The subject underwent a sigmoidectomy and left lateral hepatic sectorectomy (Surgery 1). A right-sided liver metastasis was left in place while the subject was treated with systemic chemotherapy (Chemotherapy 1). On day 120, a right hepatectomy was performed (Surgery 2). After surgery, the subject was treated for 4 months with systemic chemotherapy (Chemotherapy 2). Fig. 4B: Patient 11 had a sigmoid adenocarcinoma and two liver metastases that were treated with systemic chemotherapy before surgery (Chemotherapy 1). The subject underwent a sigmoid colectomy, left hepatic lobectomy and RFA of a solitary right hepatic lesion (Surgery 1). Imaging studies at 2 months showed recurrence in the liver, and the subject underwent a right hepatectomy (Surgery 2). Given the high risk of recurrence, chemotherapy was reinitiated (Chemotherapy 2). At 8 months, imaging showed three recurrent liver lesions and a suspicious celiac lymph node. The subject underwent RFA of these lesions and resection of the celiac node (Surgery 3). After surgery, the subject received additional chemotherapy (Chemotherapy 3); however, later imaging revealed multiple pulmonary metastases. Fig. 4A: Patient 5 underwent a left hepatectomy for recurrent disease at the time of entry into the study (day zero). Except for a questionable lung nodule in the left upper lobe, three was no evidence of disease immediately after surgery. Fifteen months later, disease recurrence was noted, with lesions found in both liver and lung.

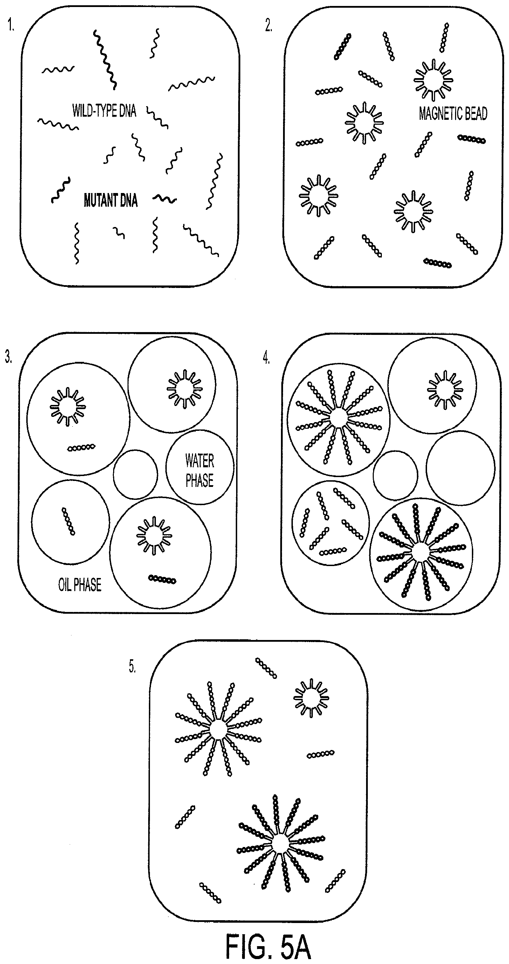

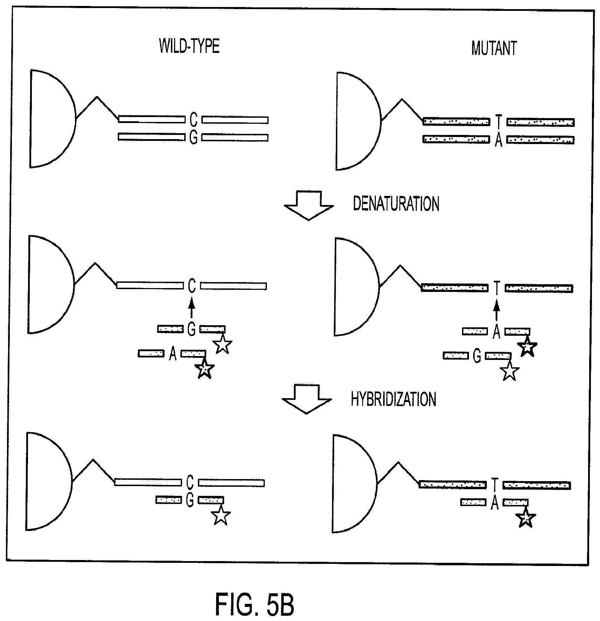

Fig. 5A-5B shows a schematic of the BEAMing-based approach for detecting mutant DNA in stool samples from patients with colorectal cancer. (Fig. 5 A) depicts the stages in the process, starting with total fecal DNA. Step 1 represents the results of sequence-specific capture of mutant and wild-type single-stranded DNA molecules. After PCR-mediated amplification of gene fragments encompassing the queried mutation sites, the DNA is mixed with magnetic beads (spheres) that are bound to oligonucleotides (spikes on the spheres) complementary to sequences in the PCR products (step 2). In Step 3, this mixture is segregated into billions of microcompartments in a water-in-oil emulsion. A small portion of these compartments contain a single bead and a single DNA template molecule, while the great majority of compartments contain neither (such as the empty bubble in the middle. When PCR is performed on these emulsions in Step 4, individual DNA fragments are amplified within the microcompartments that contain them and become covalently bound to the surface of the bead. The resultant beads are coated with tens of thousands of copies of identical DNA fragments. In Step 5, beads are recovered from the emulsion and the sequence of the bound DNA is deciphered by allele-specific hybridizion (ASH) as depicted in panel (Fig. 5 B). (Fig. 5 B) DNA amplified on magnetic microbeads by BEAMing is initially denatured to remove the non-covalently bound DNA strand. Differently labeled fluorescent probes are hybridized to the complementary target DNA covalently bound to the beads. Flow cytometry is then used to individually count beads, thereby determining the ratio of mutant to wild-type fragments originally present in the stool or plasma sample.

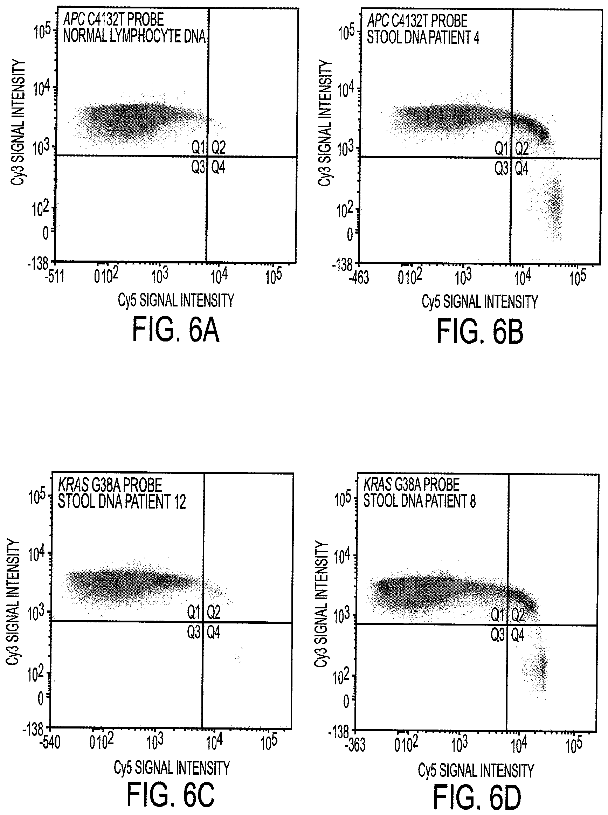

Figs. 6A-6D show scatter plot of beads analyzed by flow cytometry. BEAMing assay for APC C4132T mutation using normal lymphocyte DNA (Fig. 6A) or stool DNA from patient 4 (Fig. 6B). For lymphocyte DNA the total number of beads analyzed (all quadrants) was 253,723 with no bead containing mutant DNA (southeast quadrant, *i.e.,* quadrant 4). The total number of beads analyzed for patient 4 was 192,513, of which 747 were mutant. (Fig. 6C) BEAMing assay for KRAS G38A using stool DNA from patient 12, whose tumor did not contain this mutation. Five mutant beads were present among 305,449 analyzed beads, which were introduced by the DNA polymerase used for the initial amplification and scored as negative. (Fig. 6D) Assay of stool DNA from patient 7 whose tumor did contain a KRAS G38A. A total of 333,630 beads were analyzed, of which 685 beads were mutant.

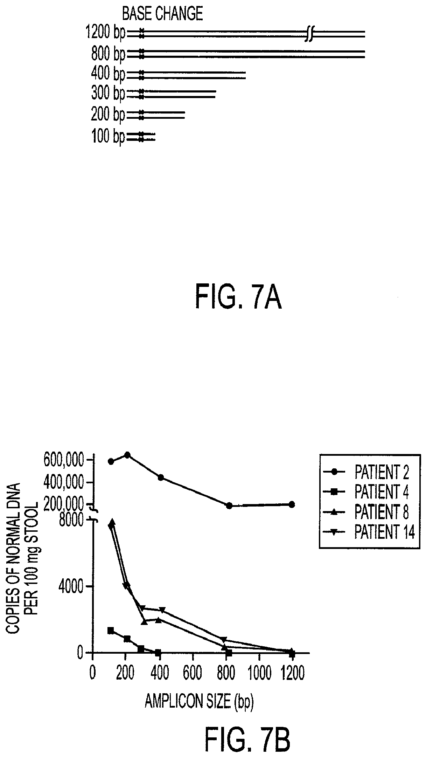

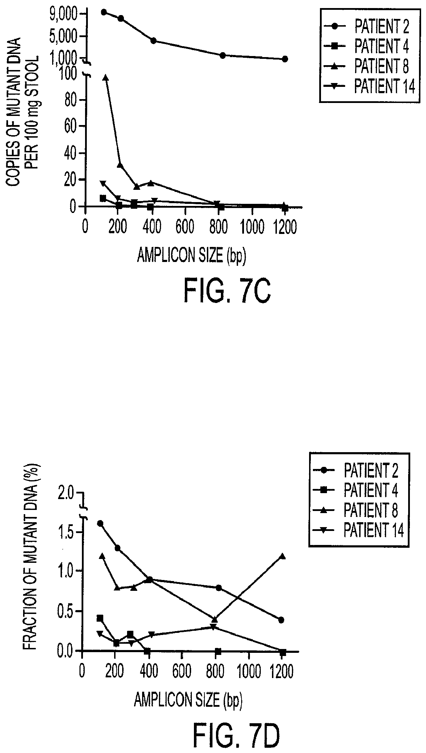

Fig. 7A-7D shows quality and quantity of normal and mutant DNA isolated from stool of patients with CRC. (Fig. 7A) Schematic of experimental design. Stool DNA was amplified with differently sized primer pairs that encompass a patient-specific DNA mutation. Real-time PCR was used to determine the total number of stool DNA fragments obtained for each amplicon size. These amplified fragments were subsequently analyzed by BEAMing to determine the number of normal Fig. 7 (B) and mutant (Fig. 7C) DNA fragments as well as the fraction of mutant to normal molecules Fig. 7 (D) present in the feces (-●- Patient 2, -■- Patient 4, **-▲-** Patient 7, -▼- Patient 14)
Fig. 8 shows mutations in fecal DNA and TNM stage. The horizontal bar shows the median fraction of mutant DNA. The whiskers represent the minimum and maximum values that were found for each indicated stage.
Fig. 9. Primers used for amplification of stool and plasma DNA. Pairs of forward and reverse primers (SEQ ID NOs: 10-53); Tag 1 (SEQ ID NO: 54); Tag 2 (SEQ ID NO: 55).
Fig. 10A-10B. Probe sequences for allele-specific hybridization, SEQ ID NOs: 56-154.
Fig. 11. Primers used for fragment sizing, SEQ ID NOs: 155-182; Tag 1, SEQ ID NO: 183.
Fig. 12. Summary of sensitivities
Fig. 13. Mutations in tumor and stool DNA determined by SBE and Sequencing
Fig. 14 shows ctDNA clearance after resection. The y-axis represents the level of ctDNA in the plasma of patient 9 The x-axis represents the time from resection, with zero as the timr of tumor removal. To calculate the half-life, a curve fit (f(t) = a - λt) based on the Marquardt-Levenberg algorithm was performed, yielding a half-life of 114 min.
Fig. 15 shows total DNA fragments in plasma prior to and after surgery. The Wisker box plot shows the total number of DNA fragments in 2 ml plasma, estimated by real-time PCR at baseline (day 0), post-surgery (day 1), day of discharge (days 2-5), and at the 1^{st} follow-up (days 13-56).
Figs. 16A-1 to 16E-3 show molecular biologic, clinical, and radiologic data on all patients in addition to those shown in Fig. 4.
Fig. 17 shows a comparison between plasma CEA and ctDNA levels in the same plasma samples. A partial residual plot comparing CEA and ctDNA levels, corrected for individual clustering, is shown. All patients' CEA and ctDNA values were used for this comparison. There was a modest overall correlation between CEA levels and ctDNA after correcting for clustering within paitents (r² = 0/2, P<0.001).
Fig. 18 shows plasma collection time-line
Fig. 19 lists the characteristics of the eighteen colorectal cancer patients evaluated.
Fig. 20 shows the 26 amplicons that were analyzed by direct sequencing. Forward primers (SEQ ID NO: 184-235). Reverse primers (SEQ ID NO: 236-287). Tag 1 (SEQ ID NO: 288). Tag 2 (SEQ ID NO: 289).

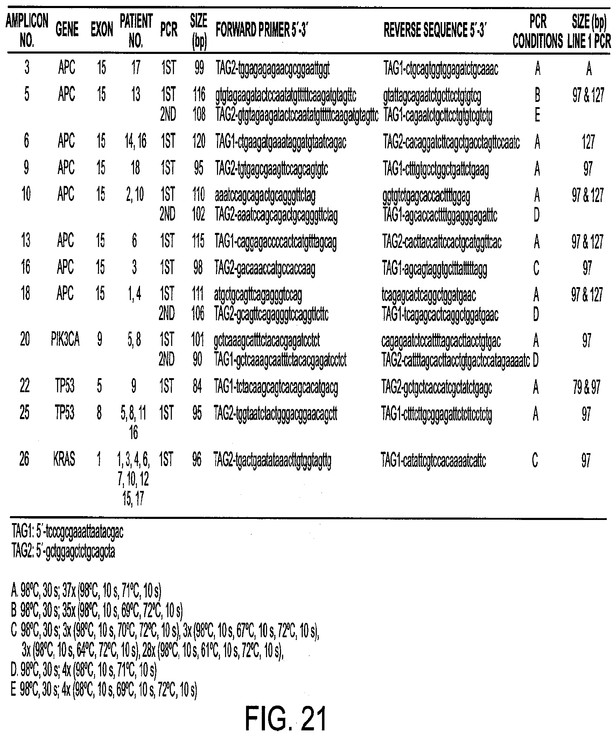

Fig. 21 shows the primers used for BEAMing each test of amplicons. Forward primers (SEQ ID NO: 290-305). Reverse primers (SEQ ID NO: 306-321). Tag 1 (SEQ ID NO: 322). Tag 2 (SEQ ID NO: 323).

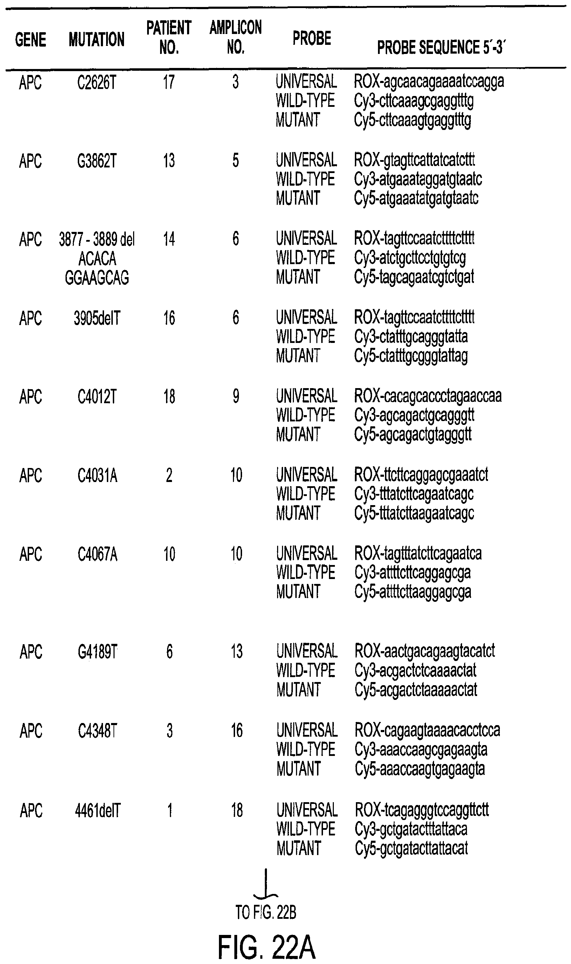

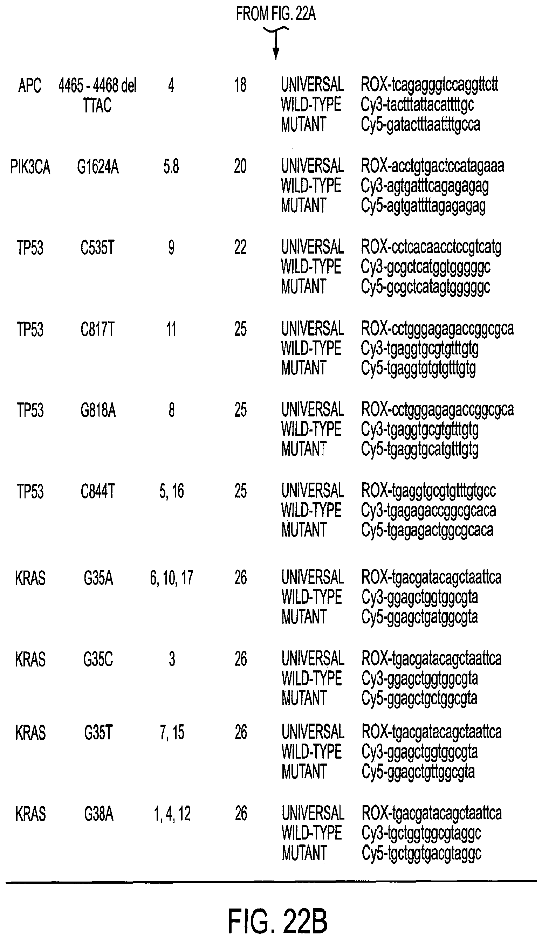

Fig. 22 shows the probes used for each test of amplicons (SEQ ID NO: 324-383, respectively.)
Fig. 23 shows patient characteristics in one of the studies
Figs. 24A-24C compare CEA and ctDNA levels for different patients

### DETAILED DESCRIPTION OF THE INVENTION

Colorectal cancer (CRC) is the second leading cause of cancer-related deaths in the United States. CRC can generally be cured by surgical excision if detected at any stage prior to distant metastasis to the liver and other organs. Unfortunately, about 35% of patients have such distant metastases, either occult or detectable, at the time of diagnosis, accounting for virtually all the deaths from the disease. The value of screening tests for colorectal neoplasia, particularly colonoscopy, has been highlighted in a variety of public awareness campaigns in the last several years. This has likely contributed to the decline in CRC-related deaths, but the large number of individuals still being diagnosed with surgically incurable cancers attests to the fact that current efforts in this regard are inadequate. In particular, there is an urgent need for non-invasive tests that can complement colonoscopy and other invasive procedures and that can be offered to patients who are hesitant to undergo such inconvenient and invasive procedures. This need has stimulated the development of new tests for early detection, including virtual colonoscopy, improved assays for the presence of blood in stool, immunohistologic tests for cancer cells or proteins in stool, and DNA-based tests for genetic or epigenetic alterations (Ouyang DL, Chen JJ, Getzenberg RH, Schoen RE. Noninvasive testing for colorectal cancer: a review. Am J Gastroenterol 2005;100:1393-403.).

Mutant DNA molecules offer unique advantages over cancer-associated biomarkers because they are so specific. Though mutations occur in individual normal cells at a low rate (∼10⁻⁹ to 10⁻¹⁰ mutations/bp/generation), such mutations represent such a tiny fraction of the total normal DNA that they are orders of magnitude below the detection limit of any test that has yet been described (including the one used in the current study). There is only one circumstance when a specific somatic mutation is present in an *appreciable* amount in any clinical sample: when it occurs in clonal fashion, *i.e.,* when the mutation is present in all cells of a specific population, thereby defining a neoplastic lesion.

Several studies have shown that mutant DNA can be detected in stool, urine, and blood of CRC patients (Osborn NK, Ahlquist DA. Stool screening for colorectal cancer: molecular approaches. Gastroenterology 2005;128:192-206). Moreover, technical factors that have limited the sensitivity of such assays are gradually being overcome. For example, improvements for stool-based testing include DNA stabilization after defecation (Olson J, Whitney DH, Durkee K, Shuber AP. DNA stabilization is critical for maximizing performance of fecal DNA-based colorectal cancer tests. Diagn Mol Pathol 2005;14:183-91.), removal of PCR inhibitors and bacterial DNA, cost-effective purification of sufficient amounts of human DNA for analysis (Whitney D, Skoletsky J, Moore K, Boynton K, Kann L, Brand R, Syngal S, Lawson M, Shuber A. Enhanced retrieval of DNA from human fecal samples results in improved performance of colorectal cancer screening test. J Mol Diagn 2004;6:386-95) and the continuing delineation of mutant genes that can be assessed (Kann L, Han J, Ahlquist D, Levin T, Rex D, Whitney D, Markowitz S, Shuber A. Improved marker combination for detection of de novo genetic variation and aberrant DNA in colorectal neoplasia. Clin Chem 2006;52:2299-302.). Moreover, assays for detecting mutations have been developed that query each template molecule individually, dramatically increasing the signal to noise ratio. Such "digital" assays are particularly well-suited for the analysis of DNA in clinical samples such as stool or plasma because the mutant DNA fragments in such samples are greatly outnumbered by normal DNA fragments.

The inventors have developed methods for monitoring tumor dynamics in cancer patients. By detection of circulating tumor DNA in the patient, predictions regarding tumor recurrence can be made. Based on the predictions, treatment and surveillance decisions can be made. For example, circulating tumor DNA which indicates a future recurrence, can lead to additional or more aggressive therapies as well as additional or more sophisticated imaging and monitoring. Circulating DNA refers to DNA that is ectopic to a tumor.

Samples which can be monitored for "circulating" tumor DNA include blood and stool. Blood samples may be for example a fraction of blood, such as serum or plasma. Similarly stool can be fractionated to purify DNA from other components. Tumor samples are used to identify a somatically mutated gene in the tumor that can be used as a marker of tumor in other locations in the body. Thus, as an example, a particular somatic mutation in a tumor can be identified by any standard means known in the art. Typical means include direct sequencing of tumor DNA, using allele-specific probes, allele- specific amplification, primer extension, etc. Once the somatic mutation is identified, it can be used in other compartments of the body to distinguish tumor derived DNA from DNA derived from other cells of the body. Somatic mutations are confirmed by determining that they do not occur in normal tissues of the body of the same patient. Types of tumors which can be monitored in this fashion are virtually unlimited. Any tumor which sheds cells and/or DNA into the blood or stool or other bodily fluid can be used. Such tumors include, in addition to colorectal tumors, tumors of the breast, lung, kidney, liver, pancreas, stomach, brain, head and neck, lymphatics, ovaries, uterus, bone, blood, etc.

Total DNA in a test sample can be determined by any means known in the art. There are many means for measuring total DNA. As detailed below, one method that can be used is a real-time PCR assay. Any gene or set of genes can be amplified. The LINE-1 gene family was employed because it is highly repeated and therefore requires a small sample to measure. The total DNA is measured so that measurements of tumor DNA collected at different times from a patient can be normalized. While genome equivalents can be used as a unit to express the total DNA content, other units of measurement can be used without limitation.

Because the amount of ectopic tumor DNA in a sample is very small, a highly sensitive means of measurement is desired. The measurement means described in detail below employs amplification on beads in an emulsion. The measurement means, called BEAMing, can detect mutations in stool and plasma DNA from patients with colorectal cancers (Fig. 5A-5B). BEAMing was named after its components - beads, emulsions, amplification, and magnetics - and essentially converts single DNA template molecules to single beads containing tens of thousands of exact copies of the template (Dressman D, Yan H, Traverso G, Kinzler KW, Vogelstein B. Transforming single DNA molecules into fluorescent magnetic particles for detection and enumeration of genetic variations. Proc. Natl. Acad. Sci. USA 2003; 100:8817-22; U.S. Serial No. 10/562,840). This method permits one to determine how frequently mutations could be detected in the DNA from plasma or stool of the same patients as well as to investigate other parameters that could be useful in designing clinically applicable DNA-based tests in the future. Other measurement means can be used, if sufficiently sensitive. The mutant sequence which is first identified in the patient's tumor DNA is assayed in the ectopic body sample, such as blood (e.g., serum or plasma), or stool. An easily collected sample is desirable. The ectopic body sample is one into which the particular type of tumor in the patient would drain. Other body samples may include saliva, broncho-alveolar lavage, lymph, milk, tears, urine, cerebrospinal fluid, etc.

The sequence that is identified as somatically mutated in the tumor DNA of the patient is specifically determined in the ectopic body sample. Similarly, the corresponding sequence that is found in the patient's other body samples is also specifically determined. Thus, for example, if a tumor mutation at nucleotide X of gene *ABC* is a G nucleotide in the tumor and a T nucleotide in other body tissues, then both the G and the T versions of nucleotide X of gene *ABC* can be specifically measured and quantified in the ectopic body sample. One means of assessing these is with allele-specific hybridization probes. Other techniques which achieve sufficient sensitivity can be used.

Calculation of the number of mutant sequences (or the ratio of mutant to not-mutant sequences) can optionally be normalized to the total DNA content, e.g., genome equivalents. The tumor burden index reflects the number of mutant (tumor) DNA molecules present in a test sample. The number of non-mutant DNA molecules in a sample may be included in the calculation of the tumor burden index to form a ratio. The normalization and/or ratio can be calculated by special purpose computer or general purpose computer or by human. The ratio can be recorded on paper, magnetic storage medium, or other data storage means. The normalized value is a data point to assess tumor burden in the whole individual. Additional assessments at different time points can optionally be made to obtain an indication of increase, decrease, or stability. The time points can be made in connection with surgery, chemotherapy, radiotherapy, or other form of therapy.

After tumor resection, if complete, a drastic decrease in tumor burden will be observed. However, if residual tumor remains, the tumor burden index will still be high or detectable. Because the half-life of ectopic DNA such as in the blood is fairly short, one can quickly assess surgical results using this technique. Incomplete resection can be detected in this means after 2 hours, 4 hours, 8 hours, 12 hours, 16 hours, 24 hours, 2 days, 3 days, 5 days, 7 days, 14 days, 21 days, 28 days, 56 days, etc. Incomplete tumor resection may lead to increased monitoring, additional surgery, additional chemotherapy, additional radiation, or combinations of therapeutic modalities. Additional therapies may include increased dosage, frequency, or other measure of aggressiveness.

Genes in which mutations can be identified are any which are subject to somatic mutation in a patient's tumor. For ease of assay development, genes which are frequently subject to such mutations may be used. These include genes which are tumor suppressors or oncogenes, genes involved in cell cycle, and the like. Some commonly mutated genes in cancers which may be used are *APC, KRAS, TP53,* and *PIK3CA.* This list is not exclusive.

While any means of detection of mutations can be used, hybridization to allele-specific nucleic acid probes has been found to be effective. Prior to hybridization, double stranded hybridization reagents are typically heated to denature or separate the two strands, making them accessible to and available for hybridization to other partners. Slow 'cooling, i.e., at least as slow as 1 degree C per second, at least as slow as 0.5 degree C per second, at least as slow as 0.25 degree C per second, at least as slow as 0.1 degree C per second, or at least as slow as 0.05 degree C per second, has been found useful. In addition, the presence of the reagent tetramethyl ammonium chloride (TMAC), has also been found to be useful, especially when one of the hybridization partners is attached to a bead.

Our results show that ctDNA is a promising biomarker for following the course of therapy in patients with metastatic colorectal cancer. ctDNA was detectable in all subjects before surgery, and serial blood sampling revealed oscillations in the level of ctDNA that correlated with the extent of surgical resection. Subjects who had detectable ctDNA after surgery generally relapsed within 1 year. The ctDNA seemed to be a much more reliable and sensitive indicator than the current standard biomarker (CEA) in this cohort of subjects.

Our studies are consistent with others that have shown that ctDNA can be detected in subjects with cancer, particularly in advanced tumors⁶. However, most such previous studies have not used techniques sufficiently sensitive to detect the low levels of ctDNA found in many of the subjects evaluated in the current study. Moreover, one of the crucial and distinguishing features of our approach lies in the ability to precisely measure the level of ctDNA rather than to simply determine whether or not ctDNA is detectable.

The results of our study suggest that ctDNA levels reflect the total systemic tumor burden, in that ctDNA levels decreased upon complete surgery and generally increased as new lesions became apparent upon radiological examination. However, whether ctDNA levels are exactly proportional to systemic tumor burden cannot be definitively determined, because there is no independent way to measure systemic total burden at this time. Radiographs are inaccurate, because lesions that are observed upon imaging are composed of live neoplastic cells, dead neoplastic cells and varying amounts of non-neoplastic cells (stromal fibroblasts, inflammatory cells, vasculature, and the like)¹¹. The proportion of these cell types in any lesion is unknown. Additionally, micrometastatic lesions that are smaller than a few millimeters, which in aggregate may make a large contribution to the total tumor burden, are not detectable by positron emission tomography, computed tomography or magnetic resonance imaging scans.

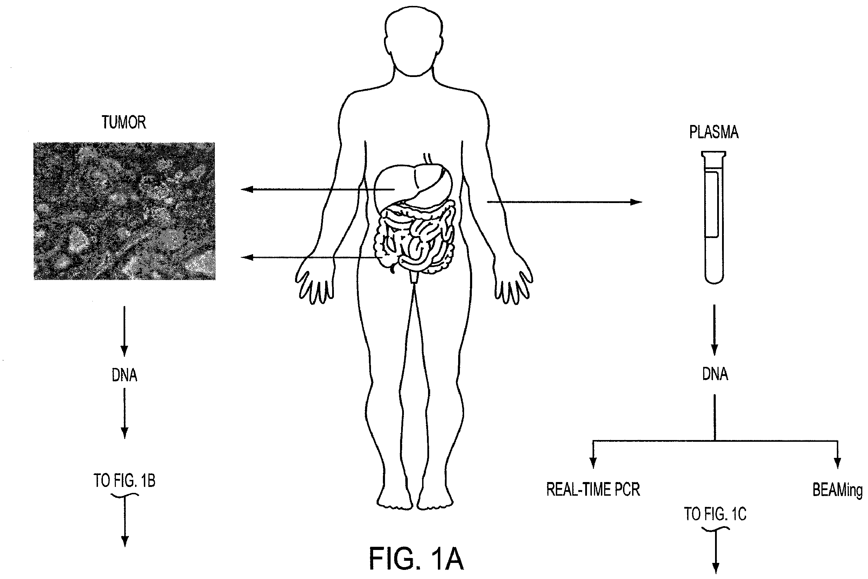

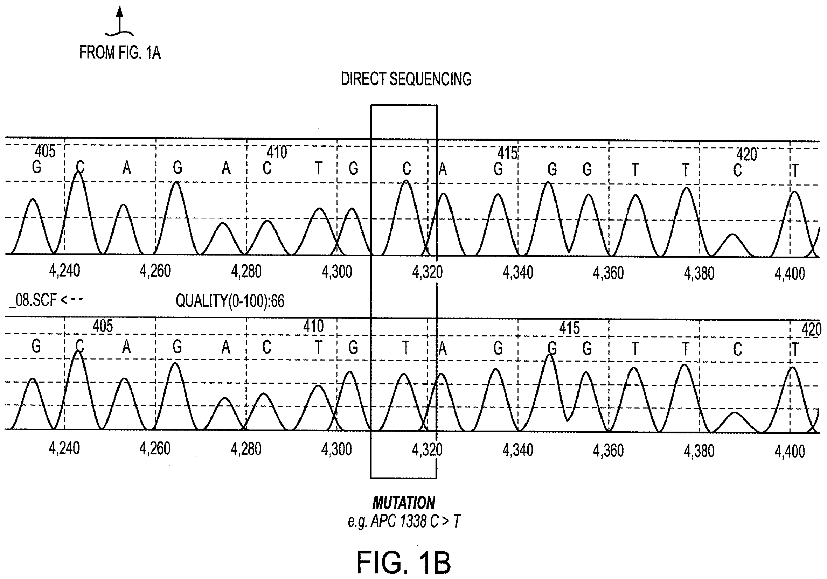

The approach used in our study can be considered a form of "personalized genomics." As such, it has both advantages and disadvantages. The advantage over other biomarkers lies in its specificity, as the queried mutation should never be found in the circulation unless residual tumor cells are present somewhere in the subject's body. The disadvantage is that a marker specific for each subject must be developed. This entails the identification of mutations in the subject's tumor as a preliminary step (Figs. 1A-1B). Though we have performed this step with direct sequencing of DNA from paraffin-embedded tissues, it could be performed with simpler technologies, such as microarrays querying mutation hotspots^{12,13}. The second step-designing and testing a mutation-specific probe-is also time consuming at this stage of technological development. But it, too, could be simplified, in that a stock of probes, representing the most common mutations, could easily be prepared in advance. This strategy may also be particularly useful for a different application of the approach, *i. e.,* cancer screening in a healthy population where mutational status is not known in advance.

In sum, we present a framework for using circulating tumor DNA as a measure of tumor dynamics. The rationale is similar to that employed in the care of patients with HIV, in whom viral nucleic acids are quantitatively assessed to monitor asymptomatic disease and used to tailor therapy to the individual's needs. We envision that ctDNA could be used to noninvasively monitor many types of cancer and, as in the treatment of individuals with HIV, help influence clinical decision-making. As sequencing technologies improve, it will become relatively simple to identify such mutations in virtually any cancer. Indeed, such diagnostic applications are one of the major goals of the Cancer Genome Atlas project.

The above disclosure generally describes the present invention. A more complete understanding can be obtained by reference to the following specific examples which are provided herein for purposes of illustration only, and are not intended to limit the scope of the invention.

### EXAMPLE 1

### METHODS

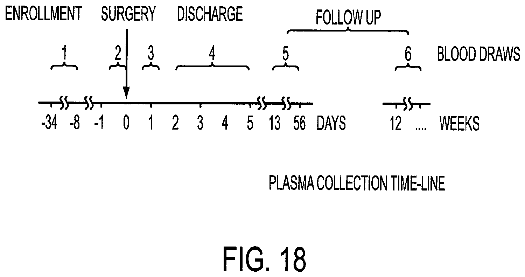

**Subjects and study design.** This study was approved by the Institutional Review Board of the Johns Hopkins Medical Institutions. Subjects were eligible if they had primary or metastatic colorectal cancer that was being treated surgically at The Johns Hopkins Sidney Kimmel Comprehensive Cancer Center. Between October 2005 and July 2006, 31 subjects diagnosed with colorectal cancer were screened during preoperative evaluation for possible surgery. Twenty-eight subjects consented for the study, but seven of these were found not to be candidates for therapy, two subjects were lost during follow-up and one subject was found to have a medical condition other than colorectal cancer, leaving eighteen participants. Each subject agreed to have ctDNA assessed in plasma samples obtained before and after surgery and during prespecified intervals during their post-operative course **(****Fig. 18****)** through October 2007. We prospectively collected 162 plasma samples from the 18 subjects. Formalin-fixed paraffin-embedded tumor tissue was obtained from each subject and processed by the Surgical Pathology Laboratory at The Johns Hopkins Medical Institutes using routine procedures. We performed the analyses of the tumor tissues and the plasma samples in a blinded fashion once the clinical assessment was complete. We measured tumor sizes radiographically with computed tomography, and we used cross-sectional measurements in centimeters to estimate tumor burden.

**Isolation and quantification of DNA from plasma.** We drew peripheral blood into EDTA tubes (Becton Dickinson). Within one hour, we subjected the tubes to centrifugation at 820g for 10 min. We transferred 1-ml aliquots of the plasma to 1.5-ml tubes and centrifuged at 16,000g for 10 min to pellet any remaining cellular debris. We transferred the supernatant to fresh tubes and stored them at -80 °C. We purified total genomic DNA from 2 ml of the plasma aliquots using the QIAamp MinElute virus vacuum kit (Qiagen) according to the manufacturer's instructions. We quantified the amount of total DNA isolated from plasma with a modified version of a human LINE-1 quantitative real-time PCR assay, as described previously¹⁴. Details are provided in

**Mutation analysis of DNA from tumor tissue.** We determined the mutation status of four genes in DNA purified from paraffin-embedded tumor tissue. We cut 10-µm sections and stained them with H&E. We used laser-capture microdissection to acquire neoplastic cells from these sections. We digested the dissected material overnight with proteinase K (Invitrogen) and purified genomic DNA from it with the QIAamp Micro Kit (Qiagen). We analyzed a total of 26 PCR products by direct sequencing. Further details concerning DNA amplification and sequencing are provided in Example 6.

**Mutation analysis of DNA from plasma.** We queried at least one mutation identified by sequencing of each subject's tumor tissue in plasma. In brief, we designed primers that could amplify the region containing the mutation for an initial amplification step with a high-fidelity DNA polymerase (New England BioLabs). We used the amplified product as a template in the subsequent BEAMing assay. The sequences of the primers and probes used for each test are listed in Example 6. The basic experimental features of BEAMing have been previously described^{l5}, and the modifications used in the current study are described in Example 6. We used the DNA purified from 2 ml plasma for each BEAMing assay. We repeated each measurement at least two times.

We used DNA purified from each subject's tumor as a positive control. We also included negative controls, performed with DNA from subjects without cancer, in each assay. Depending on the mutation being queried, the percentage of beads bound to mutant-specific probes in these negative control samples varied from 0.0061% to 0.00023%. This fraction represented sequence errors introduced by the high-fidelity DNA polymerase during the first PCR step, as explained in detail previously¹⁶. To be scored as positive in an experimental sample, the fraction of beads bound to mutant fragments had to be higher than the fraction found in the negative control, and the mean value of mutant DNA fragments per sample plus one standard deviation had to be >1.0. We analyzed bead populations generated by BEAMing at least twice for each plasma sample.

**Carcinoembryonic antigen measurement.** We analyzed CEA abundance by a two-step chemiluminescent microparticle immunoassay with the Abbott ARCHITECT i2000 instrument (Abbott Laboratories) at the Johns Hopkins Medical Institutions Clinical Chemistry Research Laboratory.

**Statistical analyses.** We quantified post-operative changes in ctDNA as a mean percentage decrease after surgery, with its standard error. We compared relative changes in CEA to ctDNA values with Student's unpaired *t*-test. We assessed changes from baseline with a one-sample *t*-test. The correlation between CEA and ctDNA levels was calculated with partial residuals from linear regression, taking into account within-patient clustering. Recurrence was defined on the basis of radiographic and clinical findings. We calculated all confidence intervals at the 95% level. We performed computations were performed using JMP 6.0 software (SAS Institute) and SigmaPlot 10.0.1 (Systat Software).

### EXAMPLE 2

### Measurement of ctDNA

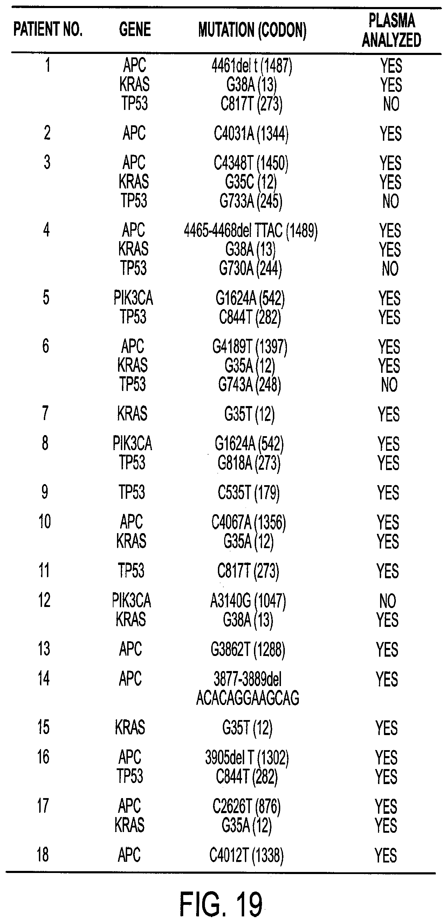

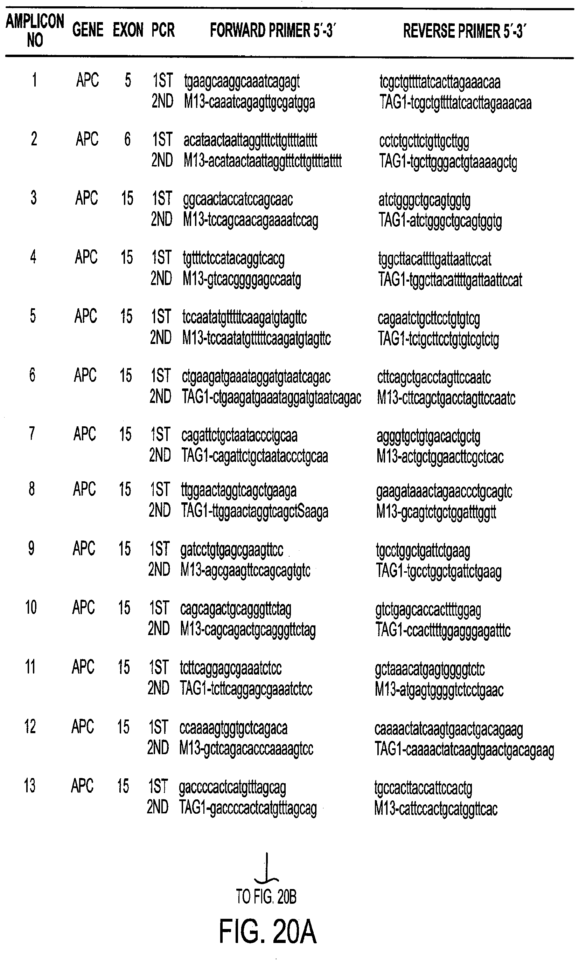

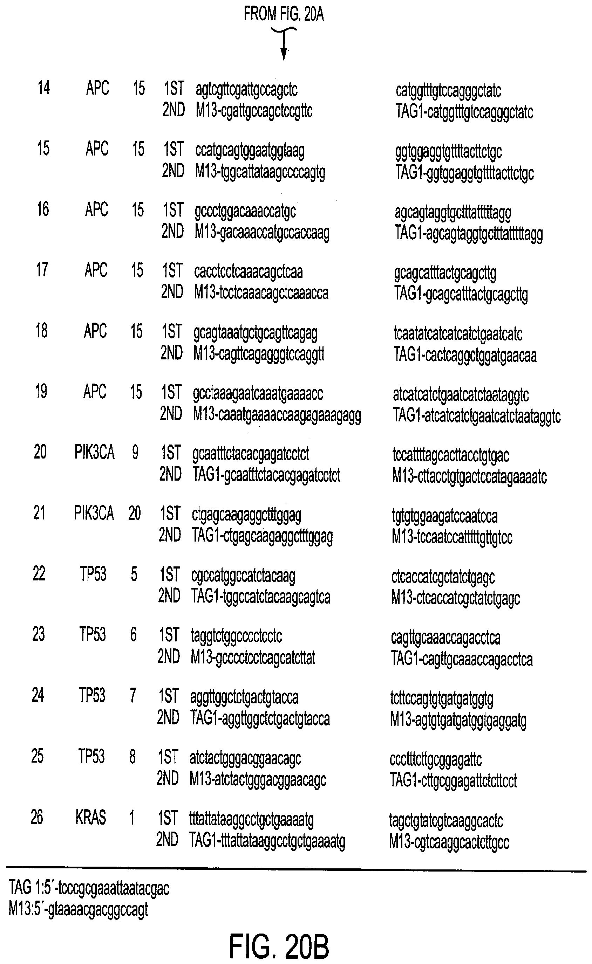

Quantification of circulating mutant ctDNA by BEAMing represents a personalized approach for assessing disease in subjects with cancer. The first step in this process is the identification of a somatic mutation in the subject's tumor **(****Fig. 1A****-1B).** Fig. 19 lists the characteristics of the subjects with colorectal cancer evaluated in this study. Four genes were assessed by direct sequencing in tumors from 18 subjects, and each of the tumors was found to have at least one mutation **(****Fig. 20****).**

The second step in the process is the estimation of the total number of DNA fragments in the plasma by real-time PCR **(****Fig. 1A****-1C).** Before surgery (day 0), there was a median of 4,000 fragments per milliliter of plasma in the 18 subjects described above (range between 10th and 90th percentiles, 1,810-12,639 DNA fragments ml⁻¹).

The third and final step is the determination of the fraction of DNA fragments of a given gene that contains the queried mutation. Such mutant DNA fragments are expected to represent only a small fraction of the total DNA fragments in the circulation. To achieve the sensitivity required for detection of such rare tumor-derived DNA fragments, we developed an improved version of BEAMing (detailed in Example 6). These improvements achieved high signal-to-noise ratios and permitted detection of many different mutations via simple hybridization probes under identical conditions. We attempted to design 28 assays, at least one for each of the 18 subjects, and were successful in every case. The median percentage of mutant DNA fragments in the 95 positive samples evaluated in this study was 0.18% (range between 10th and 90th percentiles, 0.005-11.7%). Examples of typical assays from plasma serially collected from a representative subject are shown in **Figure 2A****-2D.**

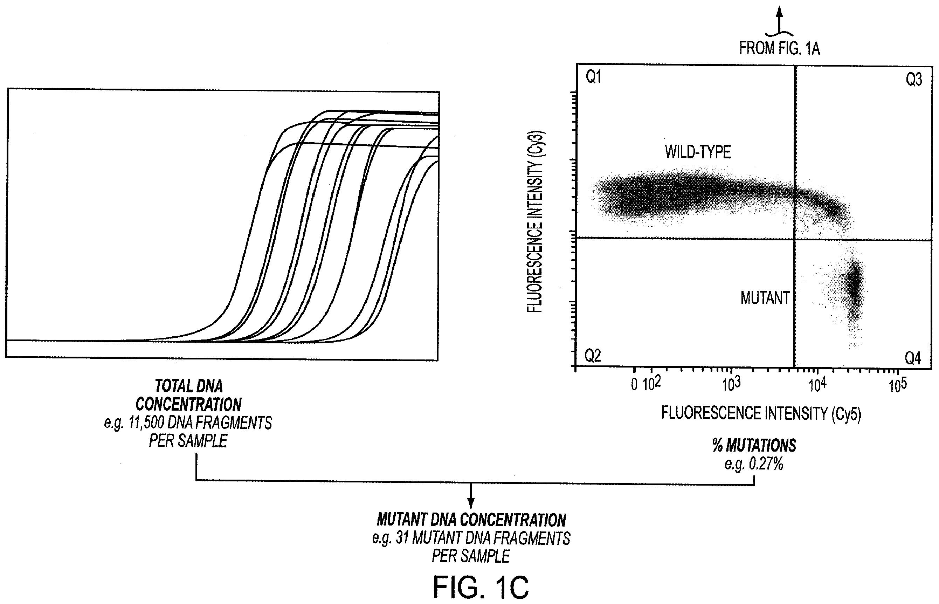

Multiplying the total number of DNA fragments of a gene in the analyzed volume of plasma (as determined by real-time PCR) by the fraction of mutant fragments (as determined by BEAMing) yields the number of mutant fragments (ctDNA number) in that volume of plasma **(****Fig. 1C****).** The median number of mutant DNA fragments in the 95 positive samples evaluated in this study was 39 (range between 10th and 90th percentiles, 1.3-1833.0).

The accuracy of these assays was assessed by measurements of the number of mutant DNA fragments derived from two different genes in the same subject. We were able to assay mutations in two different genes in 43 samples derived from nine study subjects. The ctDNA levels corresponding to the two mutant genes were found to be remarkably similar (correlation coefficient *R*² = 0.95, **Fig**. **14****)**.

### EXAMPLE 3

### ctDNA dynamics in subjects with cancer undergoing therapy

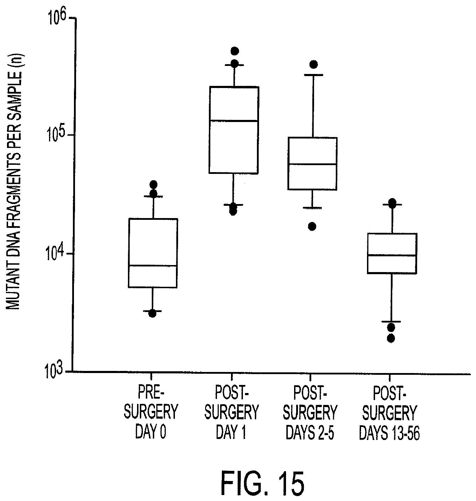

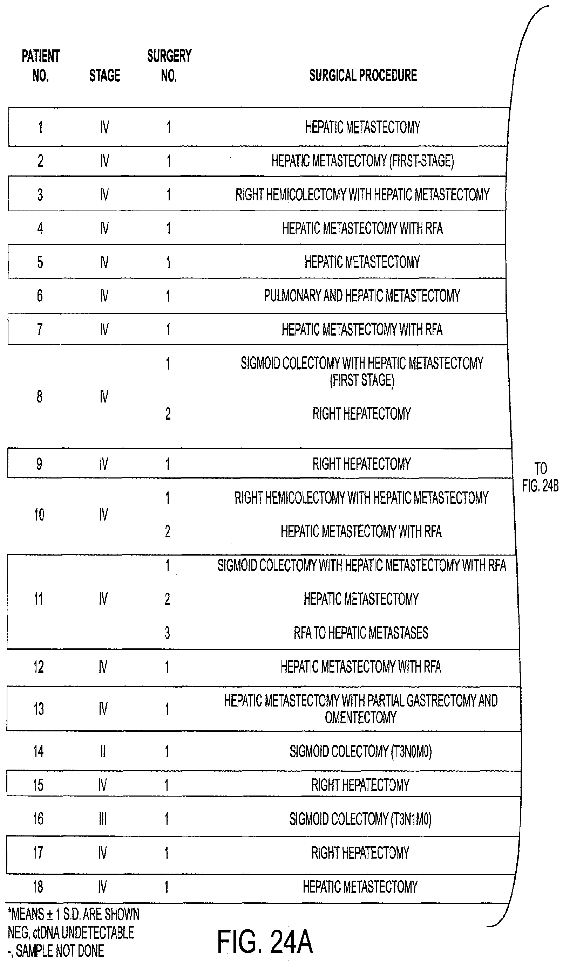

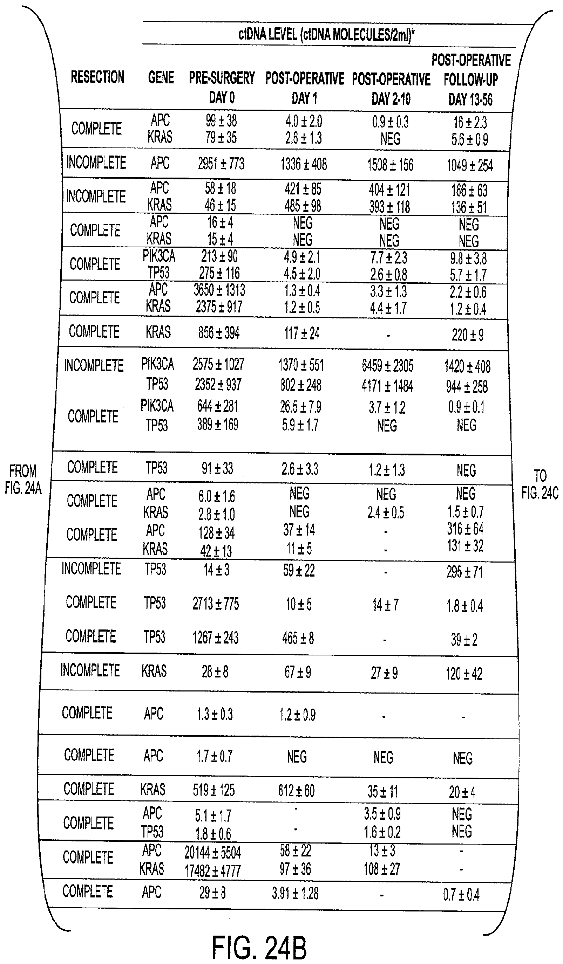

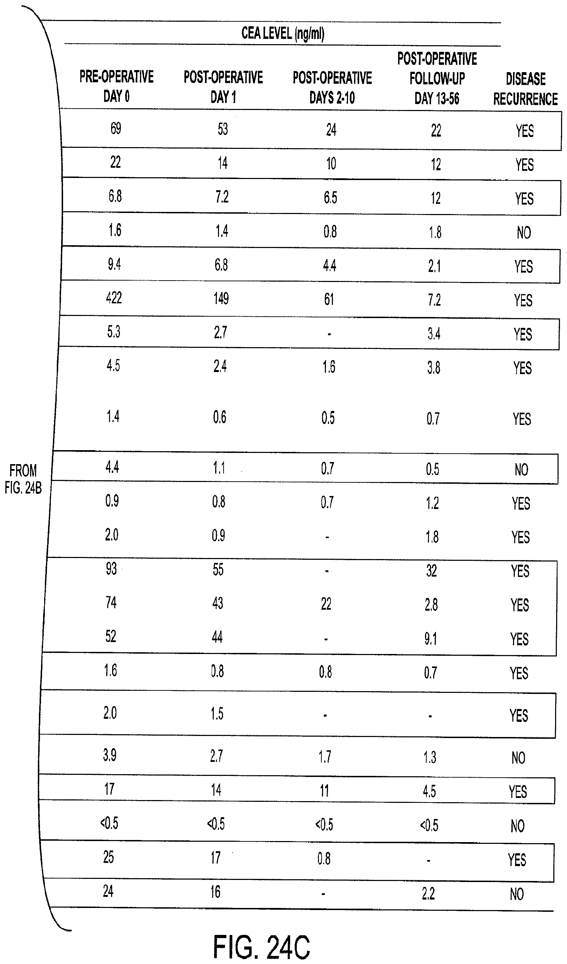

We evaluated 18 subjects after a total of 22 surgeries during the course of this study **(****Fig. 19****).** The ctDNA level determined before surgery (day 0) varied widely, ranging from 1.3 to 23,000 mutant templates per sample (median 99 mutant templates per sample; range between 10th and 90th percentiles, 3-2,837). Seventeen of these surgeries involved complete resection of all evident tumor tissue, whereas five were incomplete resections. A sharp drop in the ctDNA level by the day of discharge (two to ten days after surgery) was observed in all subjects who underwent complete resections, with a 99.0% median decrease in ctDNA (range between 10th and 90th percentiles, 58.9-99.8%; **Fig. 24A-****24C).** This decrease was already evident 24 h after surgery (96.7% median decrease, range between 10th and 90th percentiles, 31.4-100.0%). Through evaluation of a subject whose plasma was sampled at multiple early times after complete resection, we estimated the half-life of ctDNA after surgery as 114 min **(****Fig. 15****).**

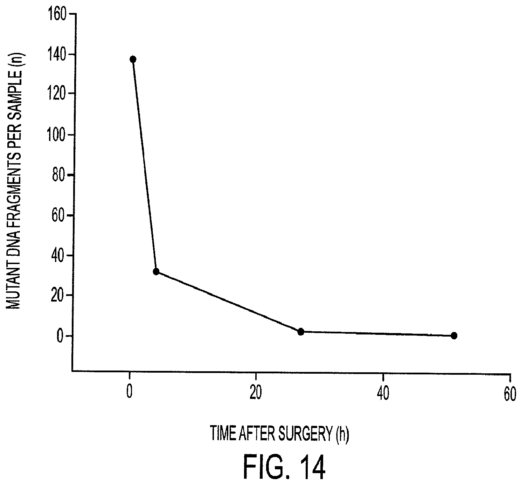

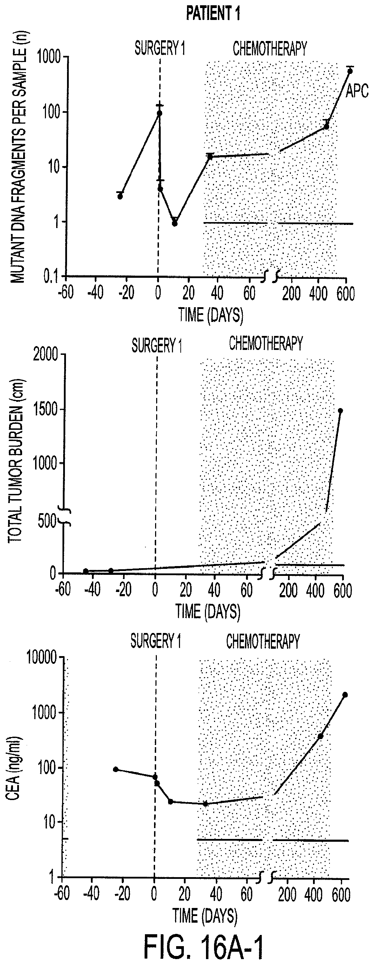

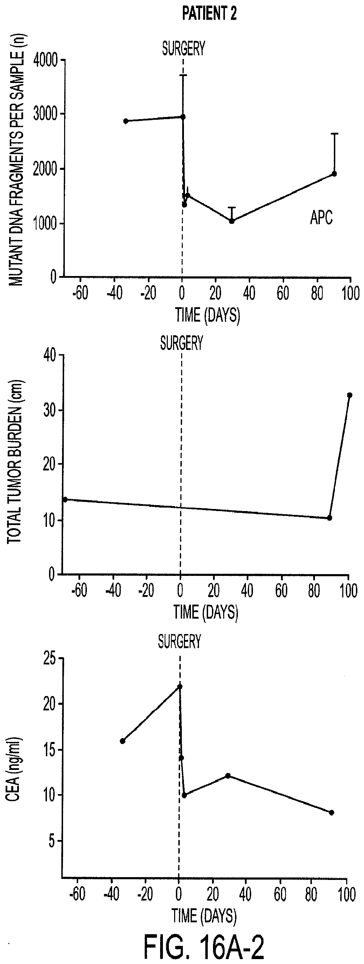

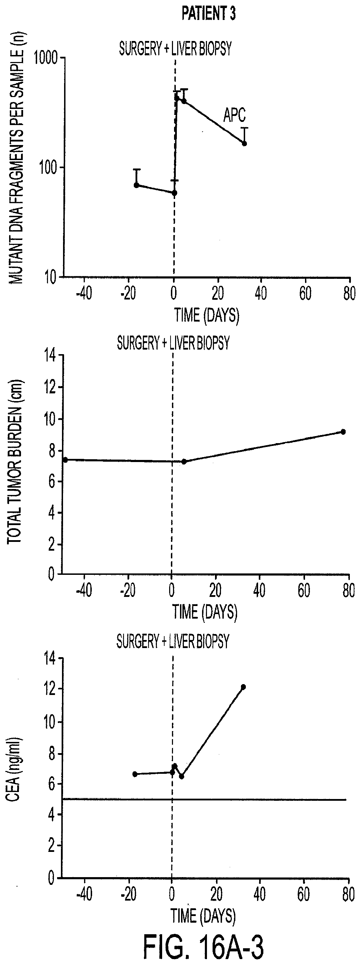

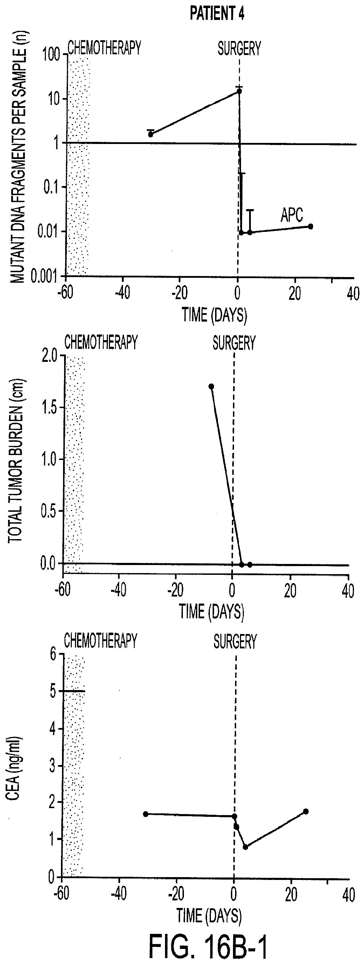

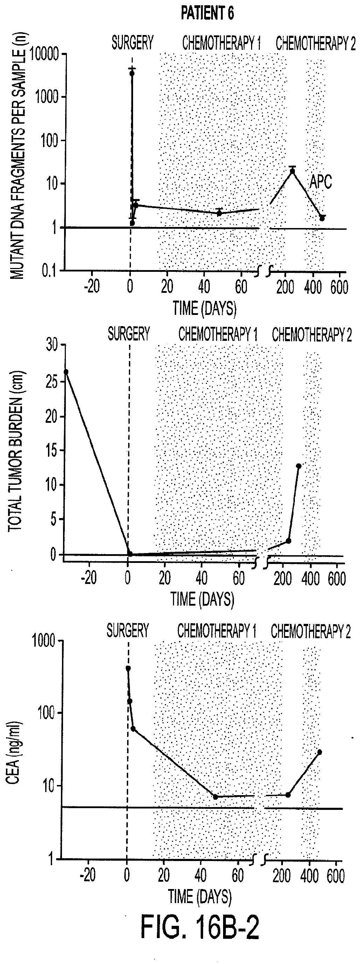

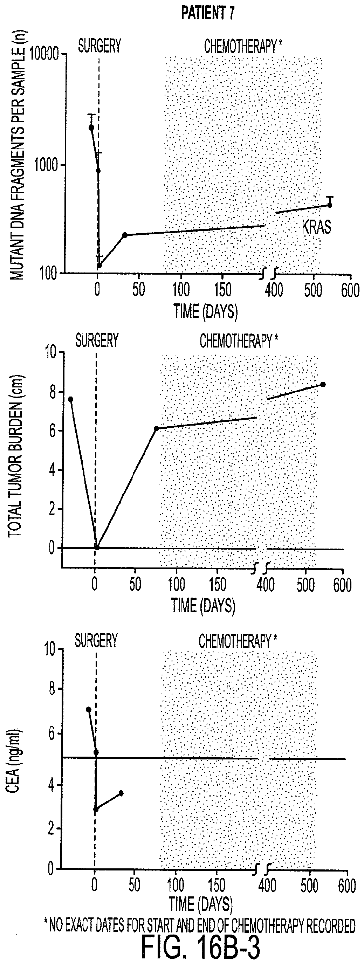

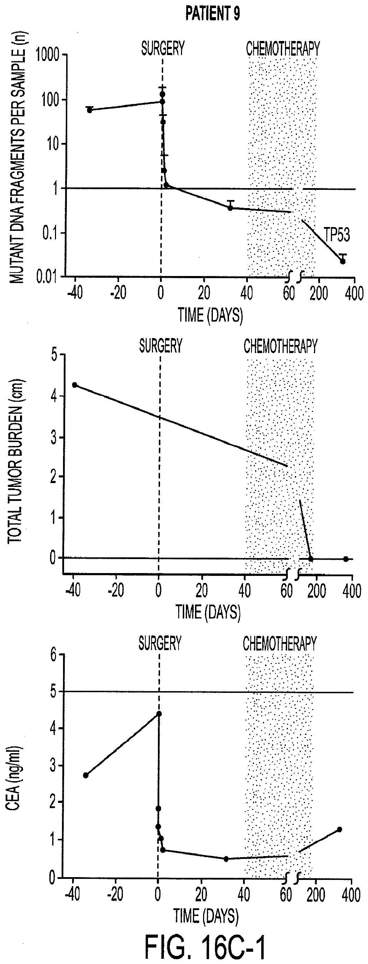

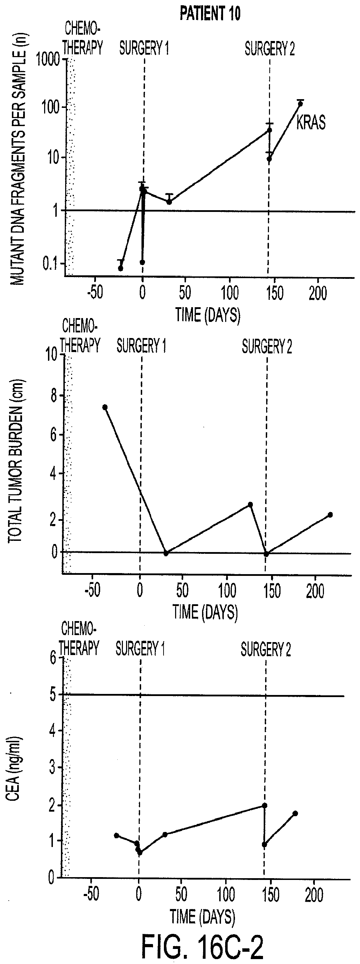

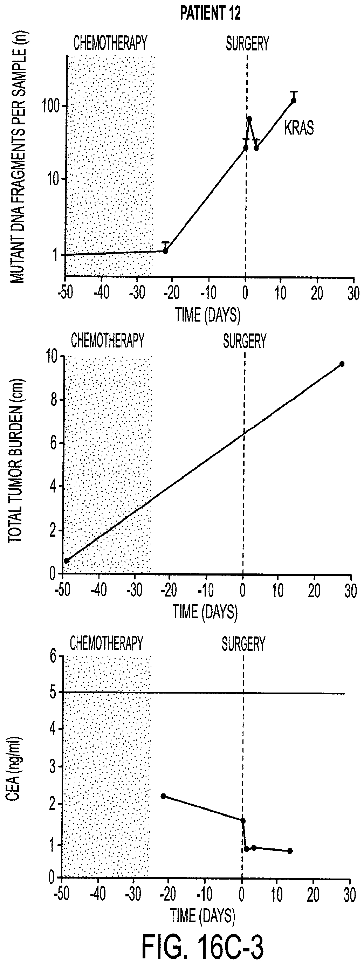

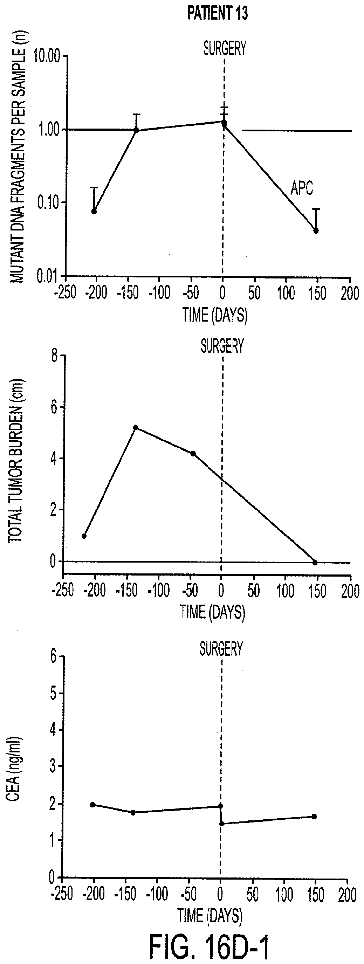

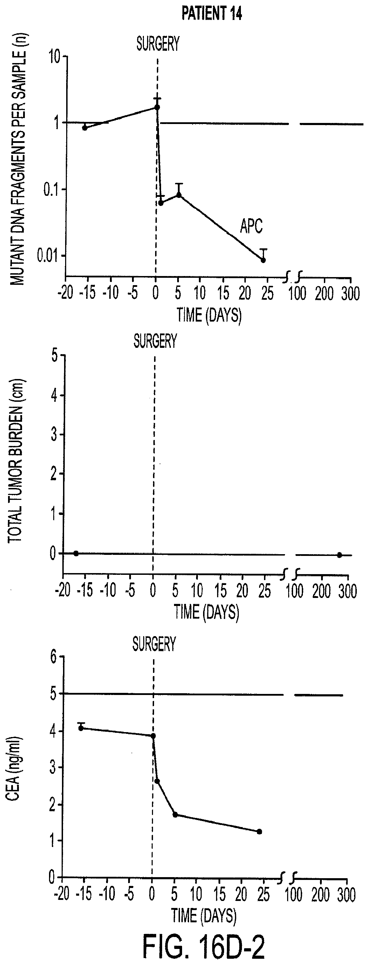

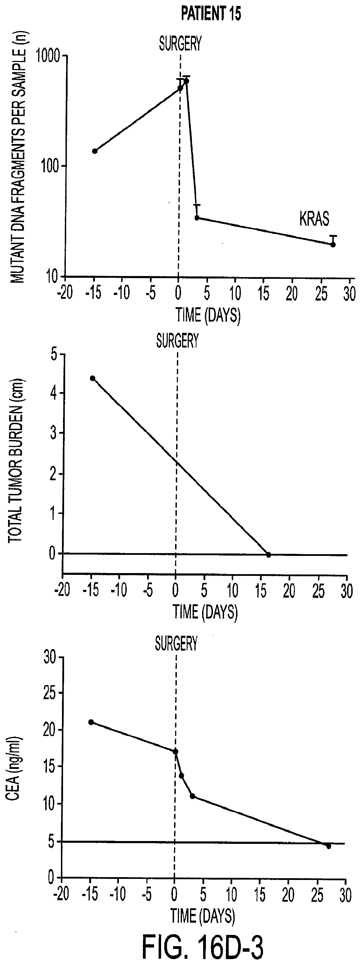

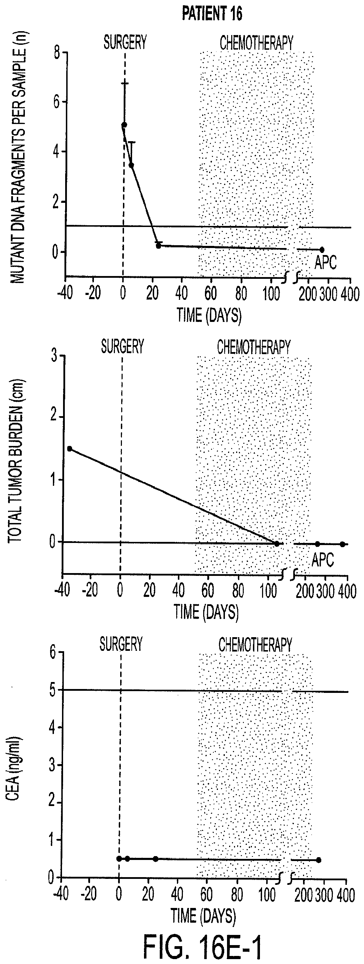

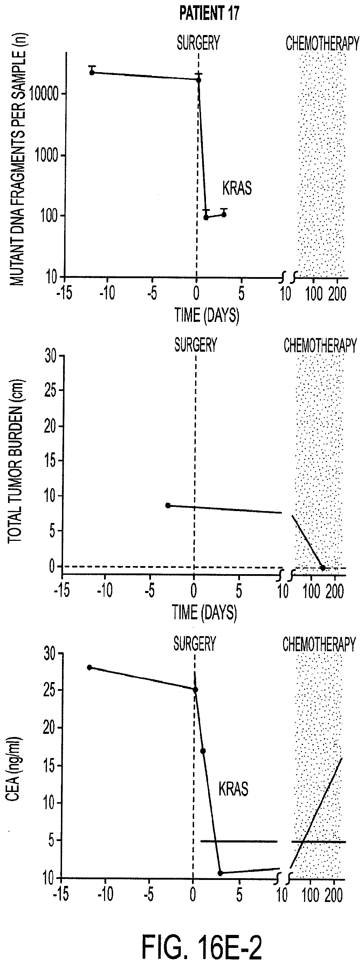

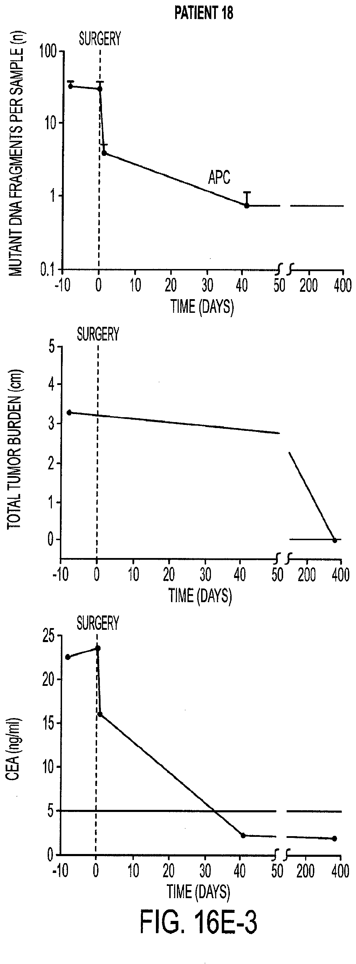

In the five cases with incomplete resections, the change in ctDNA was quite different. In two of these cases, the number of mutant fragments decreased only slightly at 24 h (55-56%), whereas in the other three cases, the number actually increased (141%, 329% and 794%). This increase was perhaps due to injury of remnant tumor tissue during the surgical procedure, with subsequent release of DNA. Surgically induced tissue injury is consistent with the observation that the total amount of DNA in the plasma (mutant plus normal) increased immediately after surgery in all subjects **(****Fig. 16A-1 to 16E-3****).**

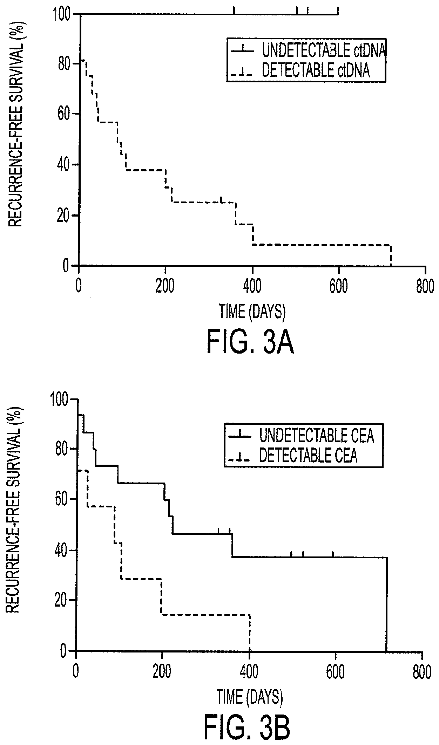

Though the amount of ctDNA generally decreased after surgery, it did not decrease to undetectable levels in most cases. Plasma samples were available from the first follow-up visit, 13-56 d after surgery, in 20 instances. ctDNA was still detectable in 16 of these 20 instances, and recurrences occurred in all but one of these 16 **(****Fig. 24A****-24C).** In a marked contrast, no recurrence occurred in the four subjects in whom ctDNA was undetectable at the first follow-up visit. **(****Fig. 24 A-24C****).** The difference in recurrence rate between subjects with and without detectable ctDNA at the first follow-up was significant (P = 0.006 by Mantel-Cox log-rank test, **Fig. 3a****).**

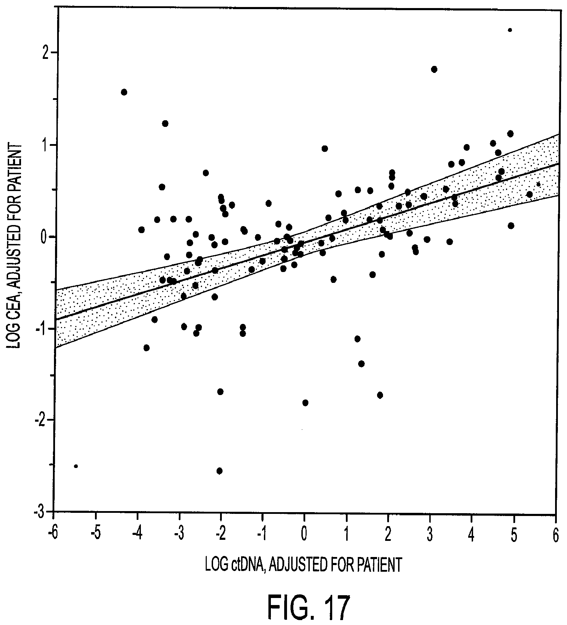

Representative time courses of ctDNA along with clinical and radiologic data on two subjects are provided in **Figure 4B****-4C,** and similar data on all other subjects are shown in **Fig. 17****.** Subjects 8 and 11 had more than one surgical procedure during the study, providing special opportunities to assess changes in ctDNA after repeated, controlled manipulation of tumor burden. Both of these subjects had incomplete resections in their initial surgery, and their ctDNA levels did not decrease **(****Fig. 4B****-4C).** They had complete resections in their second surgery, and the ctDNA abundance dropped precipitously thereafter. The ctDNA abundance then climbed back to higher levels over the next several months **(****Fig. 4B****-4C).**

Eleven of the subjects in our cohort received chemotherapy during the course of the study. In three of these subjects, ctDNA levels declined during the treatment. An example is provided by subject 8: ctDNA decreased by more than 99.9%, whereas tumor volume (composed of live and dead neoplastic cells in addition to stromal cells) decreased only slightly **(****Fig. 4A****-4C).** In six subjects, there was an immediate rise in ctDNA after discontinuation of chemotherapy, as is evident in subjects 8 and 11 after the first chemotherapy **(****Fig**. **4A****-4C)** and in subjects 1, 4, 10, and 12 **(****Fig. 17****).**

### EXAMPLE 4

### Comparison with carcinoembryonic antigen

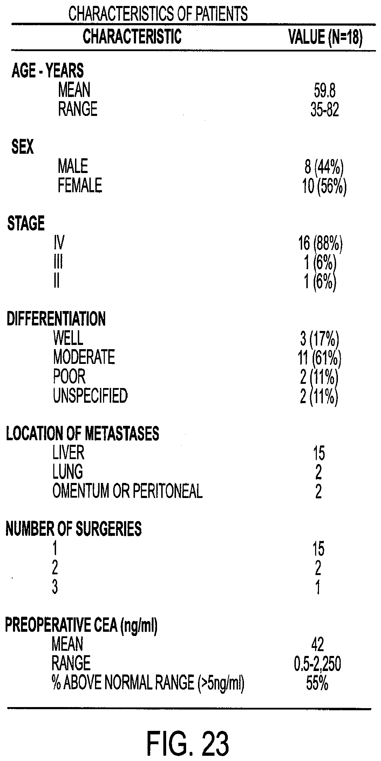

Carcinoembryonic antigen (CEA) is the standard biomarker for following disease in subjects with colorectal cancer and is routinely used in the management of the disease¹⁰. Only ten of the eighteen subjects had CEA levels >5 ng ml⁻¹ (the boundary of the normal range) before study entry. **(****Fig. 23****).** This difference in sensitivity between the two assays (ctDNA versus CEA) was statistically significant; 56% versus 100%, respectively (P = 0.008, McNemar test). Moreover, even in those subjects with positive CEA levels before surgery, complete tumor resection resulted in a much less marked decrease in CEA than that observed with ctDNA (median decrease of 99.0% versus 32.5% in ctDNA versus CEA, respectively; P < 0.001, Student's *t*-test). There was a modest overall correlation between CEA abundance and ctDNA levels after correcting for clustering within subjects (*R*² = 0.20, *P* < 0.001, **Fig. 17**). Finally, when measured at the first post-operative follow-up visit on days 24-48, the ability of CEA levels to predict recurrent disease was less impressive than that of ctDNA levels (P = 0.03 by Mantel-Cox log-rank test, **Fig**. **3b**)**.**

### EXAMPLE 5

### Study design and collection of clinical samples

For this study, specimens from subjects with colorectal cancer who had been acquired through a previous study were evaluated⁷. Subjects were at average risk for CRC as determined by family history and had no personal history of any type of cancer. Patients with non-specific abdominal symptoms or a history of basal cell or squamous cell carcinoma of the skin were not excluded. Stool and blood specimens were collected 6-12 days post-colonoscopy and prior to any bowel preparation for subsequent surgery. This study included 25 of the 40 previously identified cancer cases⁷ as 15 cases had inadequate amounts of residual material available. Patient characteristics are summarized in Table 1: Seven of the patients had stage I carcinomas, seven had stage II, eight had stage III, two had stage IV and one was of unspecified stage. The blood samples were drawn in BD Vacutainer tubes with EDTA (Becton Dickinson, Franklin Lakes, NJ USA) from 16 of the 25 patients. Plasma was prepared by centrifugation of blood at 1380 g for 30 min. The supernatant was transferred to a fresh tube and re-centrifuged. After centrifugation, the plasma was transferred to a Millipore Ultrafree-MC 0.45 micron filter device (Millipore, Billerica, MA, USA) to remove remaining cellular debris. The filter device was subjected to centrifugation at 1380 g for 15 min. The cleared plasma was transferred to a new tube and stored at -20° C until processed.

### Identification of mutations in tumor tissue

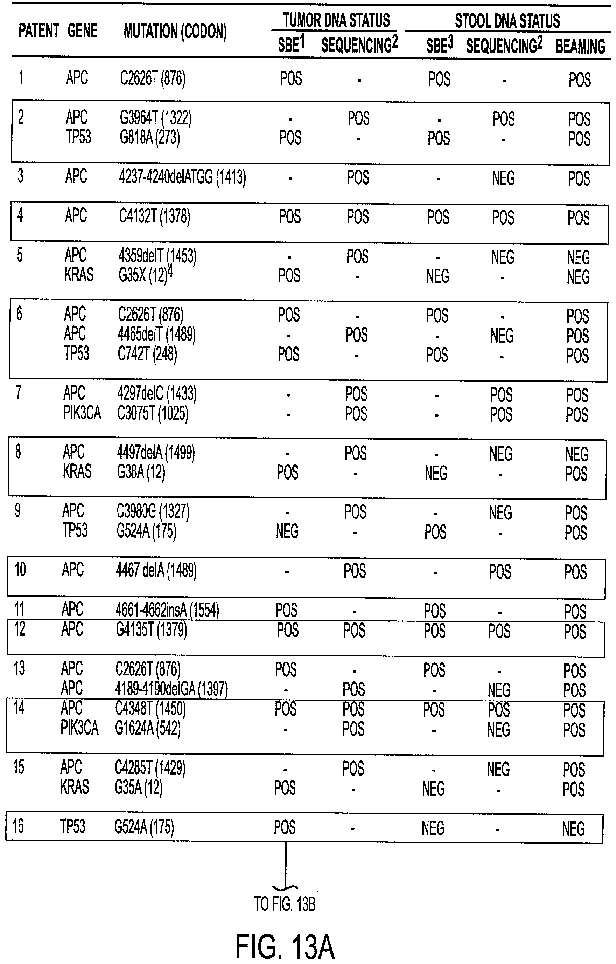

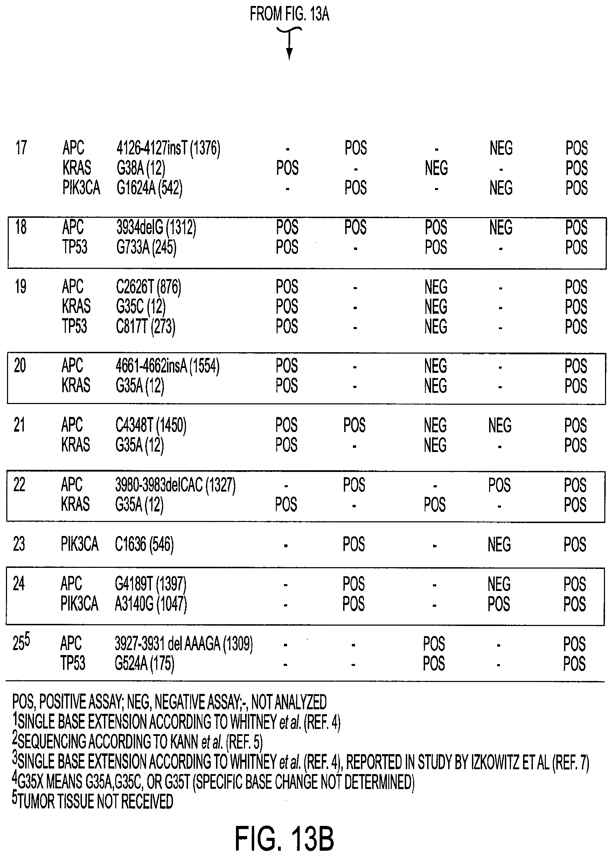

Tissues obtained upon surgical resection were used for mutation analysis, as reported previously^{5,4}. Briefly, snap-frozen or paraffin-embedded microdissected tumor tissue was used for the isolation of tumor DNA using the QIAamp DNA mini kit (Qiagen, Valencia, CA). All DNA *samples* were analyzed for 22 common mutations in *APC, TP53,* and *KRAS* using a single base extension (SBE) assay and a sequencing approach for exon 9 and 20 of *PIK3CA,* exon 3 of *CTNNB1,* and exon 15 of *APC.* The sequencing was performed by using single-stranded DNA templates in four separate sequencing reactions, each containing a R110 labeled AcyloTerminator nucleotide (PerkinElmer) and a mixture of ThermoSequenase (GE) and AcycloPol (PerkinElmer). Combined, the two marker panels were able to identify at least one mutation in the 24 tumor samples available for this study (Table 2). The sensitivity of SBE and sequencing was 75% (18/24) and 79% (19/24), respectively (Fig. 13).

### Isolation and quantification of stool DNA

Human DNA enriched for the target genes (*APC, TP53, KRAS,* and *PIK3CA*) was purified from total stool DNA using a Reversible Electrophoretic Capture Affinity Protocol (RECAP)⁸.

The copy number of gene fragments recovered from each stool sample was quantified using an iCycler™ IQ real-time PCR detection system (Biorad, Hercules, CA, USA). Duplicate reactions (50 µl) consisted of 5 µl of DNA, 10× PCR buffer (Takara Bio; Madison, WI, USA), 0.2 mM dNTPs (Promega, Madison, WI, USA), 0.5 µM of sequence-specific primers (sequences available upon request) and 2.5 U LATaq DNA polymerase (Takara Mirus Bio, Madison, WI, USA). The PCR parameters were 95°C for 3.5 min for denaturation followed by 40 cycles of 95° C for 1 min, 55° C for 1 min, and 72° C for 1 min.

### DNA isolation and quantification of plasma DNA

DNA was purified from 2 ml plasma using the QIAamp MinElute Virus Vacuum Kit (Qiagen) as recommended by the manufacturer. The DNA was eluted in EB buffer (Qiagen), and stored at -20°C. The amount of total DNA isolated from plasma was quantified using a modified version of a human LINE-1 quantitative real-time PCR assay, as described previously⁹. Details are provided in Example 6.

### Mutation analysis by BEAMing

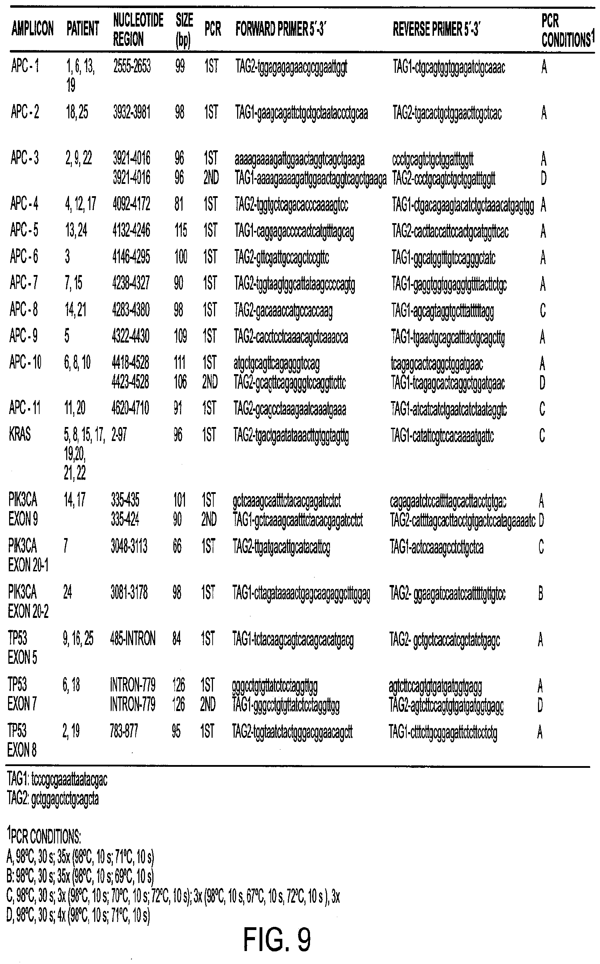

Plasma and stool DNA was analyzed for somatic mutations by BEAMing. In total, 18 amplification primer sets were designed for the analysis of 33 different mutations. For each stool sample, a total of 30,000 genome equivalents were analyzed. One genome equivalent was defined as 3.3 pg of genomic DNA and is equivalent to the DNA amount present in a haploid cell. A volume corresponding to the DNA purified from 2 ml of plasma was used for each BEAMing assay. The initial amplification was performed in multiples of 50 µl PCR reactions, each containing template DNA equivalent to 250 µl of plasma or 3,750 genome equivalents of stool DNA. Each reaction consisted of 5× Phusion high fidelity buffer, 1.5 U of Hotstart Phusion polymerase (both NEB), 0.2 µM of each primer, 0.25 mM of each dNTP, and 0.5 mM MgCl₂. Nested PCR reactions were performed for selected target regions; for the second amplification, 2 µl of the first PCR was added to a 20-µl PCR reaction of the same makeup as described above except that different primers were used. Primer sequences and cycling conditions are listed in Fig. 9. PCR products were pooled, diluted, and quantified using the PicoGreen dsDNA assay (Invitrogen). The BEAMing procedure has been described previously¹⁰ and modifications used in the current study are described here.

A LSR II flow cytometry system (BD Bioscience) equipped with a high throughput autosampler was *used* for the analysis of each bead population. On average, 5 × 10⁶ beads were analyzed for each plasma sample. The flow cytometric data was gated so that only single beads with extension products (as indicated by the control probe) were used for analysis. The mutation frequency was calculated as the number of gated beads attached to mutant sequences divided by the number of beads containing either mutant or wild-type sequences. In order for an assay to be scored as positive, it had to meet two criteria. First, the fraction of mutant beads had to be higher than the background emanating from polymerase errors arising during amplification. We used a Poison distribution to estimate the expected variation in the background observed with DNA templates derived from normal lymphocyte DNA. A "positive" assay was scored as one in which the fraction was higher than 0.01 %. The second criterion was that the calculated number of mutant sequences in the templates used for analysis had to be ≥ 1. For example, if in a sample, only 1,000 genomic equivalents were analyzed, yet the calculated fraction of beads bound to mutant sequences was 0.05 % (1 in 2,000), this sample was scored as negative as the number of mutant template molecules was only 0.5 (0.05% x 1,000), which is less than 1.

### Detection of Somatic Mutations by BEAMing

We assessed the performance of BEAMing for the detection of 33 different base changes in either *APC* (20), *KRAS* (4), *PIK3CA* (4), or *TP53* (5). The BEAMing procedure was performed as described previously with the important exception that an allele-specific hybridization (ASH) approach was developed for the analysis of bead-bound DNA (Fig. 1A-1C). The hybridization was performed with equimolar concentrations of fluorescently-labeled oligonucleotides complementary to the immobilized wild-type or mutant DNA sequences. Optimal allele discrimination for all 33 base changes was reached by an initial denaturation step followed by a slow cooling process in a tetramethylammonium chloride (TMAC) based buffer¹¹. All mutations we attempted to assess (transitions, transversions, insertions or deletions ranging from 1 to 5 bp) were successfully detected, with high signal to noise ratios, using this single TMAC-based hybridization procedure.

An example of ASH applied to beads generated by BEAMing is shown in Fig. 6. Positive control DNA populations were prepared using long oligonucleotides representing the genomic sequences, with the mutations in the center. Negative controls were prepared from DNA isolated from lymphocytes of healthy donors. Because no polymerase is completely error-free, mutations introduced during the initial amplification step create a small number of beads with mutant DNA sequences even when no mutant *DNA* templates are present in the sample DNA¹². In the current study, we used Phusion DNA polymerase (NEB) because it has been shown to have the lowest error rate of any commercially available enzyme tested¹². This background was individually determined for each mutation analyzed in the current study. The median background of mutations stemming from polymerase errors in normal lymphocyte DNA was 0.0009% (range 0.01% to 0.00013%). Variations in background rates were observed between and within genes, presumably reflecting the non-random nature of polymerase errors. Accordingly, an assay for a given sample was scored "positive for mutation" only if the mutant fraction was higher than the background by a conservative and statistically significant margin (see Methods). Additionally, samples were scored as positive only if the calculated number of beads bound to mutant sequences was higher than a threshold defined by the genomic equivalents used in the assay, as also explained in more detail in the Methods.

### Quantity and Quality of the DNA purified from stool

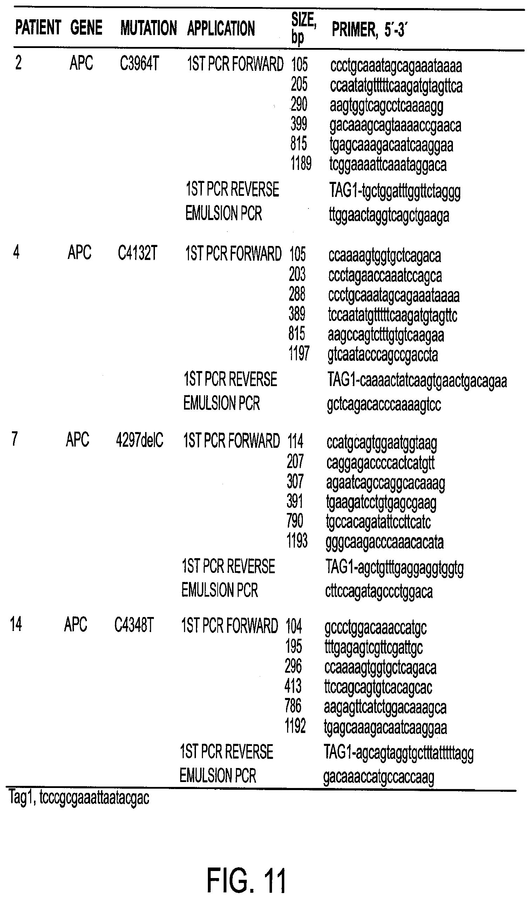

Because BEAMing cannot only be used to detect mutant DNA templates but also to precisely quantify their abundance, it could be used to determine both the quantity and quality of cell-free *mutant* and normal DNA present in the stool of CRC patients. We therefore began the current study by analyzing the sizes of the mutant DNA fragments present in the stool of CRC patients. For this purpose, six PCR primer sets were designed for the amplification of DNA fragments that encompassed different *APC* mutations found in four patients with localized colorectal cancers. Two of the patients harbored Stage I and two harbored Stage II cancer (Fig. 11). The amplicon sizes obtained with these primers varied between 104 bp and 1,197 bp, with the mutations located in the middle of each amplicon (Fig. 7A). The DNA purified from an equal mass (181 mg) of stool was used in each assay. The number of mutant or normal template molecules was calculated by multiplying the respective fraction of beads bound to mutant or normal DNA sequences by the DNA concentrations measured by quantitative real-time PCR. In all four patients, the number of amplifiable normal DNA fragments decreased with increasing amplicon size. In patient 2, this decrease was only 3-fold whereas the decrease was more severe - up to a thousand-fold - in the other three patients (Fig. 7B). The mutant DNA fragments decreased with size in a similar, but not identical, fashion (Fig. 7C). As a result, the fraction of mutant DNA fragments was highest in the smallest amplicons (Fig. 7D); in patients 4 and 14, we could not detect *any* mutant DNA fragments when the largest amplicon size (1200 bp) was employed. These findings were important as they suggested that the sensitivity of tests for mutations in fecal DNA can be optimized by employing small amplicons. Based on this result, all the BEAMing assays used in the subsequent phases of this study were performed with ∼100 bp amplicons whenever possible, and never longer than 126 bp (Fig. 9).

### Mutation detection in stool DNA by BEAMing

The clinicopathological characteristics of the 25 patients included in this study are summarized in Table 1.

**Table 1. Patient characteristics**

| Characteristic | Value (n=25) |
|---|---|
| Age - yr | |
| Mean | 67 |
| Median (range) | 66 (50-84) |
| | |
| Gender | |
| Female | 15 |
| Male | 10 |
| | |
| Stage | |
| I | 7 |
| II | 7 |
| III | 8 |
| IV | 2 |
| unknown | 1 |
| | |
| Differentiation | |
| Well | 12 |
| Moderate | 9 |
| Poor | 3 |
| Unspecified | 1 |
| | |
| Number of mutations in tumor tissue | |
| 0 | 0 |
| 1 | 7 |
| 2 | 15 |
| 3 | 3 |

Tumors ranged in size from 12-80 mm with a mean size of 41 mm (median 40 mm). Fourteen (56%) patients were early stage (Stage I or II), 10 (40%) were late stage (Stage III or IV), and one patient was of unknown stage. As outlined in the above, of the 24 patients were tumor tissue had been available, all had at least one mutation in the primary tumor (Fig. 13). For patient 25, where no tissue was available, two mutations were identified in stool DNA by the SBE assay (Fig. 13).

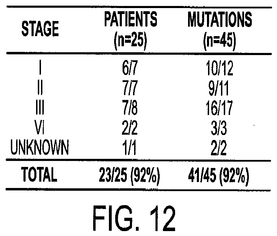

Forty-five BEAMing assays were performed to assess the 33 different mutations in these samples (13 patients had at least one mutation found in another patient; Table 2). Of the 25 patients, 23 (92%, CI: 74%, 99%) had detectable levels of mutant DNA in their stool samples. Mutations were detected as readily in patients with early stage colorectal cancers (Stages I and II) as in patients with late stage cancers (Stages III and IV) (Fig. 12). Interestingly, in one of the two patients in whom mutant DNA fragments could not be identified in the stool, the amount of normal DNA was very high (Patient 5).

The median fraction of mutant DNA present in stool samples was 0.32% but varied widely (range 0.0062% to 21.1 %; Table 2).

**Table 2:**

| | | **Tumor** | | | | | | **Stool DNA** | | | **Plasma DNA** | | |
|---|---|---|---|---|---|---|---|---|---|---|---|---|---|
| **Patient** | **Sex/age, yr** | **Stage (TNM)** | **Histology¹** | **Site²** | **Size, mm** | **Gene** | **Mutation (codon)** | **Total DNA fragments per 362 mg Stool** | **Mutant DNA , %** | **Score** | **Total DNA fragments per 2 ml Plasma** | **Mutant DNA, %** | **Score** |
| 1 | M/66 | I (T1N0M0) | Mod | R | 50 | APC | C2626T(876) | 50.600 | 4.0 | + | | | |
| 2 | F/64 | I (T2N0M0) | Mod | Sig | 30 | APC | G3964T (1322) | 398,000 | 0.87 | + | 3676 | 0.000 | - |
| | | | | | | TP53 | G818A (273) | 302,000 | 0.20 | + | | | |
| 3 | F/70 | I (T2N0M0) | Mod | C | 45 | APC | 4237-4240delATGG (1413) | 1,808 | 0.71 | + | 2397 | 1.88 | + |
| 4 | F/67 | I (T1N0Mx) | Well | Tr | 40 | APC | C4132T (1378) | 8,460 | 0.39 | + | 9317 | 0.013 | + |
| 5 | M/69 | I (T2N0M0) | Well | Rs | 24 | APC | 4359delT (1453) | 1,030,000 | 0.00 0 | - | 3365 | 0.000 | - |
| | | | | | | KRAS | G35X (12)³ | 1,030,000 | 0.00 0 | - | | | |
| 6 | M/84 | I (T2N0M0) | Mod | R | 25 | APC | C2626T (876) | 252,000 | 0.32 | + | | | |
| | | | | | | APC | 4465delT (1489) | 252,000 | 0.78 | + | | | |
| | | | | | | TP53 | C742T (248) | 252,000 | 1.0 | + | | | |
| 7 | F/58 | I (T2N0Mx) | Well | Sig | 12 | APC | 4297 delC (1433) | 13,840 | 1.0 | + | | | |
| | | | | | | PIK3CA | C3075T (1025) | 8,260 | 21 | + | | | |
| 8 | M/80 | II (T3N0Mx) | Well | Sig | 25 | APC | 4497delA (1499) | 7,420 | 0.00 3 | - | | | |
| | | | | | | KRAS | G38A (12) | 7,420 | 0.21 | + | | | |
| 9 | M/70 | II (T3N0Mx) | Well | Sf | 50 | APC | C3980G (1327) | 59,600 | 15.0 | + | 10652 | 0.002 | - |
| | | | | | | TP53 | G524A (175) | 59,600 | 0.3 | + | | | |
| 10 | F/58 | II (T3NOM0) | Well | Tr | 80 | APC | 4467 delA (1489) | 113,800 | 1.17 | + | 4530 | 0.002 | - |
| 11 | F/65 | II (T3N0M0) | Well | C | 50 | APC | 4661-4662insA (1554) | 106,600 | 1.09 | + | | | |
| 12 | F/75 | II (T3N0M0) | Well | R | 25 | APC | G4135T (1379) | 540.000 | 0.37 | + | 4650 | 0.42 | + |
| 13 | M/80 | II (T3N0Mx) | Well | As | 65 | APC | C2626T (876) | 264,000 | 0.06 | + | | | |
| | | | | | | APC | 4189-4190delGA (1397) | 264,000 | 0.04 | + | | | |
| 14 | M/66 | II (T3N0M0) | Mod | R | 45 | APC | C4348T (1450) | 15,740 | 0.2 | + | 3690 | 0.005 | - |
| | | | | | | PIK3CA | G1624A (542) | 5.340 | 0.3 | + | | | |
| 15 | F/50 | III (T4N1M0) | Mod | R | 25 | APC | C4285T (1429) | 22,600 | 0.13 | + | 6422 | 0.062 | + |
| | | | | | | KRAS | G35A (12) | 22.600 | 0.2 | + | | | |
| 16 | M/64 | III (T3N1M0) | Poor | Sig | 30 | TP53 | G524A (175) | 6,920 | 0.00 6 | - | 7047 | 0.033 | + |
| 17 | M/74 | III (T3N2M0) | Well | R | 30 | APC | 4126-4127insT (1376) | 7,140 | 0.05 9 | + | 2679 | 0.17 | + |
| | | | | | | KRAS | G38A (12) | 7,140 | 0.07 9 | + | | | |
| | | | | | | PIK3CA | G1624A (542) | 7,140 | 0.05 0 | + | | | |
| 18 | M/57 | III (T3N1M0) | Mod /Poor | Sig | 70 | APC | 3934delG (1312) | 18,700 | 0.28 | + | | | |
| | | | | | | TP53 | G733A (245) | 17,460 | 1.3 | + | | | |
| 19 | F/65 | III (T3N2Mx) | Mod/ Poor | As | 50 | APC | C2626T (876) | 5,920 | 0.18 | + | 11716 | 0.002 | - |
| | | | | | | KRAS | G35C (12) | 3,320 | 0.23 | + | | | |
| | | | | | | TP53 | C817T (273) | 3,320 | 0.10 | + | | | |
| 20 | M/59 | III (T3N1Mx) | Well | Tr | 40 | APC | 4661-4662insA (1554) | 11,320 | 0.00 62 | + | | | |
| | | | | | | KRAS | G35A (12) | 11.320 | 0.3 | + | | | |
| 21 | M/73 | III (T2N1Mx) | Mod | Tr | 42 | APC | C4348T (1450) | 10,280 | 0.05 5 | + | 5043 | 0.007 | - |
| | | | | | | KRAS | G35A (12) | 7.200 | 0.23 | + | | | |
| 22 | F/61 | III (T3N1M0) | Mod | R | NR | APC | 3980-3983delCAC (1327) | 62,800 | 7.60 | + | 4206 | 0.001 | - |
| | | | | | | KRAS | G35A (12) | 62,800 | 4.43 | + | | | |
| 23 | M/67 | IV (T3N2M1) | Mod | Sig | 60 | PIK3CA | C1636A(546) | 138,000 | 0.06 8 | + | 29233 | 6.6 | + |
| 24 | M/65 | IV (T3N1M1) | Well | As | 30 | APC | G4189T(1397) | 254,000 | 0.62 | + | 4094 | 0.44 | + |
| | | | | | | PIK3CA | A3140G (1047) | 254,000 | 1.33 | + | | | |
| 25 | M/64 | NR | NR | R | 35 | APC | 3927-3931del AAAGA (1309) | 356,000 | 0.90 | + | | | |
| | | | | | | TP53 | G524A(175) | 356,000 | 10.2 | + | | | |
| | | | | | | | | | | | | | |
| NR: not received | | | | | | | | | | | | | |
| ¹Histology type. Well: well differentiated adenocarcinoma: Mod.: moderately differentiated adenocarcinoma: Poor: Poorly differentiated adenocarcinoma. | | | | | | | | | | | | | |
| ²Location: R.: Rectum: Sip.: sigmoid colon: C.: cecum: Tr.: transverse colon; As.: ascending colon, Rs.: Rectosipmoid, Sf.: Splenic flexure | | | | | | | | | | | | | |
| ³G35X means G35A, G35C, or G35T (specific base change not determined) | | | | | | | | | | | | | |

In most cases where two mutations could be assessed in the same stool sample, the fraction of mutant DNA molecules was similar. However, in four cases (patients 7, 8, 20 and 25) there was more than a 5-fold difference in the fraction of mutant DNA fragments from one gene compared to those in another gene.

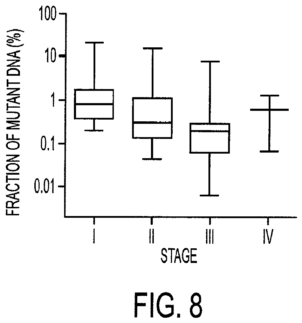

Another important observation was that the median fraction of mutant DNA fragments in stool samples did not vary significantly across the stage of the patient's tumor: 0.83%, 0.31%, 0.20%, and 0.62% for Stage I, II, III, and IV, respectively (Fig. 8).

Finally, it was of interest to compare the results of BEAMing assays in these stool samples with those obtained previously using a modified sequencing approach⁵ and single base extension (SBE)⁷ (Fig. 13)). Of the 25 patients assessed in the current study, these assays combined were able to detect at least one mutation in only 15 patients (60% of the 25 analyzed) while BEAMing detected 23 (92% of the same 25 patients). This difference was statistically significant (Table 2, p=0.008, exact McNemar's test). The SBE assay alone, which comprises the component of a commercially available DNA test that assess 22 specific mutations in *APC, TP53,* and *KRAS,* performed about as well as the sequencing-based assay (60% (12/20) vs. 56% (10/18)). Our data also revealed a potential basis for the lower sensitivity of the SBE and sequencing tests compared to BEAMing. Those mutations that *were not* detected with these tests constituted 0.11% +/-3.0% of the analyzed fragments. In contrast, those mutations that *were* detectable with SBE or sequencing were nine times more abundant (median 1.0% +/- 5.0%).

### Mutation detection in stool and plasma DNA by BEAMing

Sixteen pairs of matched samples of blood and plasma were available for analysis. For each sample, one of the mutations found in the patients' tumor was selected for analysis. As noted in Table 2, 14 of these 16 (87.5%) patients' stool samples contained mutations at detectable levels. Mutant DNA fragments were found in a smaller proportion of the plasma samples (8 of 16 [50%]; p-value for difference between the number of patients positive in *the* plasma and stool assays was 0.07 by the exact McNemar's test). There was only one patient that was negative for both tests (patient 5) and one patient with a negative stool test but a positive plasma test (patient 16). In patients that scored positive, the median fraction of mutant DNA was similar in stool (0.37%) and plasma (0.42%).

Though many previous studies have reported the presence of mutations in fecal DNA, this is the first to analyze them in a highly sensitive and quantitative manner. Similarly, other publications have reported the identification of genetic alterations in plasma or serum, but none have compared the results obtained with circulating DNA to those obtained with fecal DNA using identical techniques. The comparisons and quantifications reported here are important for guiding the development of sensitive and specific non-invasive screening tests for colorectal tumors in the future.

The quantitative analysis of fecal DNA highlighted several issues that are important for further research in this area. First, the highest sensitivities were realized when the amplicons were small, optimally less than 100 bp (Fig. 7A-7D). This is undoubtedly due to the DNA degradation that occurs either in cancer cells undergoing apoptosis or *necrosis in situ* or after they are released into the fecal stream. A similar size-dependence for DNA mutation detection has been described in plasma¹³. Note that this observation is not contradictory to studies showing that an increase in DNA integrity can be used as a marker for colorectal cancer¹⁴. Mutant DNA present in the stool of cancer patients represents only a minor fraction (median 0.32%; mean 1.89%) of the total DNA and therefore has little influence on the measurement of the integrity of the total (mutant plus normal) DNA. The observed increase of DNA integrity in cancer patients is most likely caused by the release of larger DNA fragments from normal cells within the tumor environment into the fecal stream. Indeed, recent results have shown that cancers are routinely infiltrated with particular types of inflammatory cells that could contribute relatively large DNA fragments of normal sequence ¹⁵.

Second, the results make it clear that a minimum number of DNA template molecules must be obtained to realize the sensitivity afforded by BEAMing. The sensitivity of BEAMing for any of the analyzed mutations is such that at least one mutant template can be detected among 10,000 normal templates (0.01%). For some mutations, the sensitivity is as high as one mutant template among 800,000 normal templates (0.0013%). The sensitivity is only limited by the error rate of the polymerase used in the initial amplification¹². Utilization of this high technical sensitivity in practice, however, requires an adequate number of DNA templates. For example, if only 2,000 templates molecules are used per assay, then the maximum sensitivity that can be achieved is 0.05% rather than 0.01%. Obtaining this number of templates is not problematic with stool samples, but is often problematic for plasma. In the current study, 2 ml of plasma contained a median of 4,590 genome equivalents of DNA. This may be why the plasma-based assay was less sensitive (60%) than the stool-based assay (88%) in the same patients. To routinely obtain 30,000 genome equivalents from plasma (the number employed for the stool-based tests), 50 ml of blood would be necessary. Though this may be feasible in future prospective studies, it is unlikely to be available in retrospective studies such as ours.

Though stool provides a nearly limitless supply of DNA, there are other technical issues that affect the assay results. For example, stool contains a variety of PCR-inhibitors and a large excess of bacterial DNA, necessitating sequence-specific capture of human genomic DNA. Cost-effective methods for such capture have been developed and were used in the current study. However, they have not yet been optimized for the isolation of *small* DNA fragments that contain the mutations of interest. As shown in Fig. 7A-7D, the sizes of normal and mutant DNA fragments corresponding to specific genetic regions is not necessarily the same. The fraction of mutant fragments as a function of size is likely to vary both with the particular mutation in a patient-specific manner, as it depends both on the source of the normal DNA as well as the extent of degradation of the tumor DNA fragments. This issue could have affected our results in two ways. First, it could be responsible for the wide variations among the fractions of mutant fragments observed within two different genes in some patients (e.g., Patient 7 in Table 2). Second, it could explain why we were unable to detect mutations in some patients. For example, one of these two patients (Patient 5) had a very large number of normal fragments in his stool, more than two-fold that of any other patient. Optimization of the capture probes could in the future increase sensitivity over and above the 92% obtained in the current study.

The new results also inform discussion of the relative advantages and disadvantages of stool *vs.* plasma analysis for early detection. As noted above, it is easier to obtain sufficient amounts of DNA from stool than from plasma. However, plasma is more convenient to collect from a practical standpoint, as it can be obtained during routine office visits, and it is easier to purify DNA from plasma than from stool. The sensitivity of detecting mutations in plasma from colorectal cancer patients (50%) is less than that in stool, but this could perhaps be increased by using more plasma in each assay. Perhaps the greatest advantage of stool versus plasma, however, is in the relative fractions of mutations observed in the feces of patients with different stage tumors. As shown in Figs. 8 and 12, the fraction of mutations in stool of early stage patients was as high as that in late stage patients. In contrast, our previous studies have shown that the fraction of mutations in the plasma of early stage patients is considerably lower than that of late stage patients (not apparent in the current study due to the small numbers of patients with positive plasma samples)¹³. Furthermore, the situation is likely to be even more pronounced in patients with large adenomas, as mutant DNA is much more difficult to detect in the plasma than in the stool of patients with these benign, but clinically significant, neoplasms.

Though our study represents a step towards clinical implementation of a new, more sensitive and quantitative assay than currently available commercially, several additional steps will be necessary to realize this goal. In addition to clinical studies employing large numbers of patients with varying stage colorectal tumors and equally large numbers of controls, there are still technical issues to be overcome. In particular, cost-effective methods for querying a panel of genetic markers with BEAMing must be developed. In this regard, it is notable that mutations in all 25 patients in the current study were revealed by the study of a relatively small number of common mutations. We envision that nearly 86% of patients with either colorectal cancer or large adenomas would harbor at least one of the 100 most common mutations. Implementation of such an assay would include parallel capture of∼10 exons and the subsequent multiplex PCR amplification of these DNA fragments. The newly described hybridization-based approach for mutation detection has also an advantage in that it can be easily automated. Next generation sequencing has the potential to further simplify the approach; the beads obtained by BEAMing can be analyzed by sequencing rather than by flow cytometry ¹⁶. Additionally, the mutation marker panel could be reduced in size by including epigenetic markers ¹⁷. Indeed, the lessons learned from the current study could be applied to optimize quantitative assays for methylation-based BEAMing or for any other tests for tumor- specific DNA variations that are developed in the future.

### References for EXAMPLE 5

1. Ouyang DL, Chen JJ, Getzenberg RH, Schoen RE. Noninvasive testing for colorectal cancer: a review. Am J Gastroenterol 2005;100:1393-403.
2. Osbom NK, Ahlquist DA. Stool screening for colorectal cancer: molecular approaches. Gastroenterology 2005; 128:192-206.
3. Olson J, Whitney DH, Durkee K, Shuber AP. DNA stabilization is critical for maximizing performance of fecal DNA-based colorectal cancer tests. Diagn Mol Pathol 2005;14:183-91.
4. Whitney D, Skoletsky J, Moore K, Boynton K, Kann L, Brand R, Syngal S, Lawson M, Shuber A. Enhanced retrieval of DNA from human fecal samples results in improved performance of colorectal cancer screening test. J Mol Diagn 2004; 6:386-95.
5. Kann L, Han J, Ahlquist D, Levin T, Rex D, Whitney D, Markowitz S, Shuber A. Improved marker combination for detection of de novo genetic variation and aberrant DNA in colorectal neoplasia. Clin Chem 2006; 52:2299-302.
6. Dressman D, Yan H, Traverso G, Kinzler KW, Vogelstein B. Transforming single DNA molecules into fluorescent magnetic particles for detection and enumeration of genetic variations. Proc Natl Acad Sci U S A 2003;100:8817-22.; U.S. Serial No. 10/562,840
7. Itzkowitz SH, Jandorf L, Brand R, Rabeneck L, Schroy PC, 3rd, Sontag S, Johnson D, Skoletsky J, Durkee K, Markowitz S, Shuber A. Improved fecal DNA test for colorectal cancer screening. Clin Gastroenterol Hepatol 2007;5:111-7.
8. Kent Moore J, Smith JA, Whitney DH, Durkee KH, Shuber AP. An electrophoretic capture method for efficient recovery of rare sequences from heterogeneous DNA. Biotechniques 2008;44:363-74.
9. Rago C, Huso DL, Diehl F, Karim B, Liu G, Papadopoulos N, Samuels Y, Velculescu VE, Vogelstein B, Kinzler KW, Diaz LA, Jr. Serial Assessment of Human Tumor Burdens in Mice by the Analysis of Circulating DNA. Cancer Res 2007;67:9364-9370.
10. Diehl F, Li M, He Y, Kinzler KW, Vogelstein B, Dressman D. BEAMing: single-molecule PCR on microparticles in water-in-oil emulsions. Nat Methods 2006;3:551-9.
11. Wood WI, Gitschier J, Lasky LA, Lawn RM. Base composition-independent hybridization in tetramethylammonium chloride: a method for oligonucleotide screening of highly complex gene libraries. Proc Natl Acad Sci U S A 1985;82:1585-8.
12. Li M, Diehl F, Dressman D, Vogelstein B, Kinzler KW. BEAMing up for detection and quantification of rare sequence variants. Nat Methods 2006;3:95-7.
13. Diehl F, Li M, Dressman D, He Y, Shen D, Szabo S, Diaz LA, Jr., Goodman SN, David KA, Juhl H, Kinzler KW, Vogelstein B. Detection and quantification of mutations in the plasma of patients with colorectal tumors. Proc Natl Acad Sci U S A 2005;102:16368-16373.
14. Boynton KA, Summerhayes IC, Ahlquist DA, Shuber AP. DNA integrity as a potential marker for stool-based detection of colorectal cancer. Clin Chem 2003;49:1058-65.
15. Galon J, Costes A, Sanchez-Cabo F, Kirilovsky A, Mlecnik B, Lagorce-Pages C, Tosolini M, Camus M, Berger A, Wind P, Zinzindohoue F, Bruneval P, Cugnenc PH, Trajanoski Z, Fridman WH, Pages F. Type, density, and location of immune cells within human colorectal tumors predict clinical outcome. Science 2006;313:1960-4.
16. Shendure J, Porreca GJ, Reppas NB, Lin X, McCutcheon JP, Rosenbaum AM, Wang MD, Zhang K, Mitra RD, Church GM. Accurate multiplex polony sequencing of an evolved bacterial genome. Science 2005;309:1728-32.
17. Chen WD, Han ZJ, Skoletsky J, Olson J, Sah J, Myeroff L, Platzer P, Lu S, Dawson D, Willis J, Pretlow TP, Lutterbaugh J, Kasturi L, Willson JK, Rao JS, Shuber A, Markowitz SD. Detection in fecal DNA of colon cancer-specific methylation of the nonexpressed vimentin gene. J Natl Cancer Inst 2005;97:1124-32.

### EXAMPLE 6

### Isolation of DNA from formalin-fixed, paraffin embedded (FFPE) tumor tissue

Eighteen tumor specimens were collected after liver or colon surgery, fixed in formalin, and embedded in paraffin. Ten µm sections were cut and mounted on PEN-membrane slides (Palm GmbH, Bernried, Germany). The sections were deparaffinized and stained with hematoxylin and eosin. All specimens underwent histological examination to confirm the presence of tumor tissue, which was dissected from completely dried sections with a MicroBeam laser microdissection instrument (Palm). The dissected tumor tissue was digested overnight at 60°C in 15 µl ATL buffer (Qiagen) and 10 µl Proteinase K (20 mg/ml; Invitrogen). DNA was isolated using the QIAamp DNA Micro Kit (Qiagen) following the manufacturer's protocol. The isolated DNA was quantified by hLINE-1 quantitative PCR as described below.

### PCR amplification and direct sequencing of DNA isolated from tumor tissue

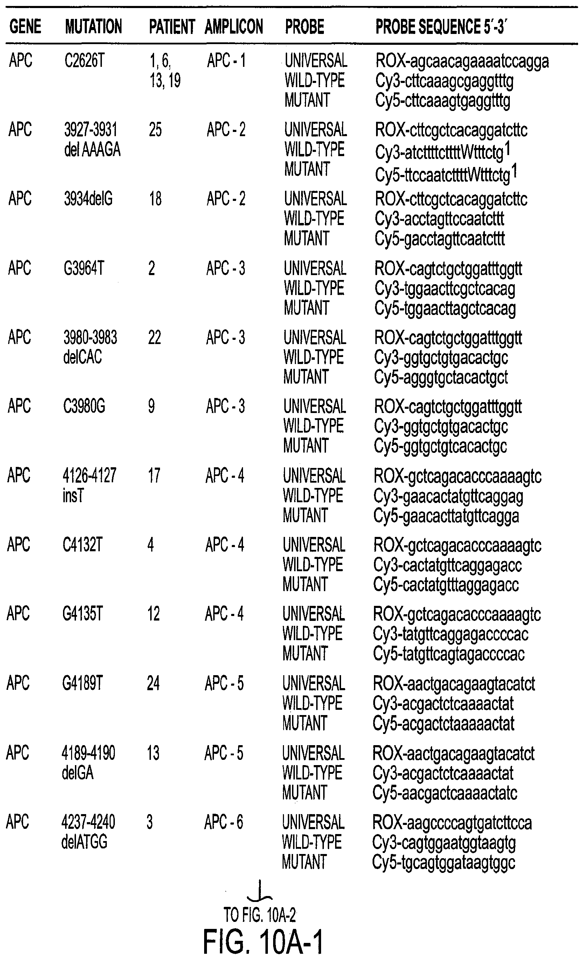

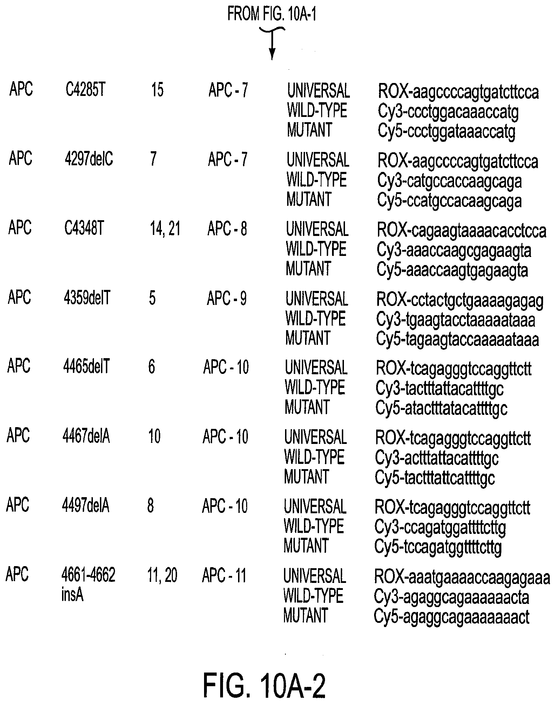

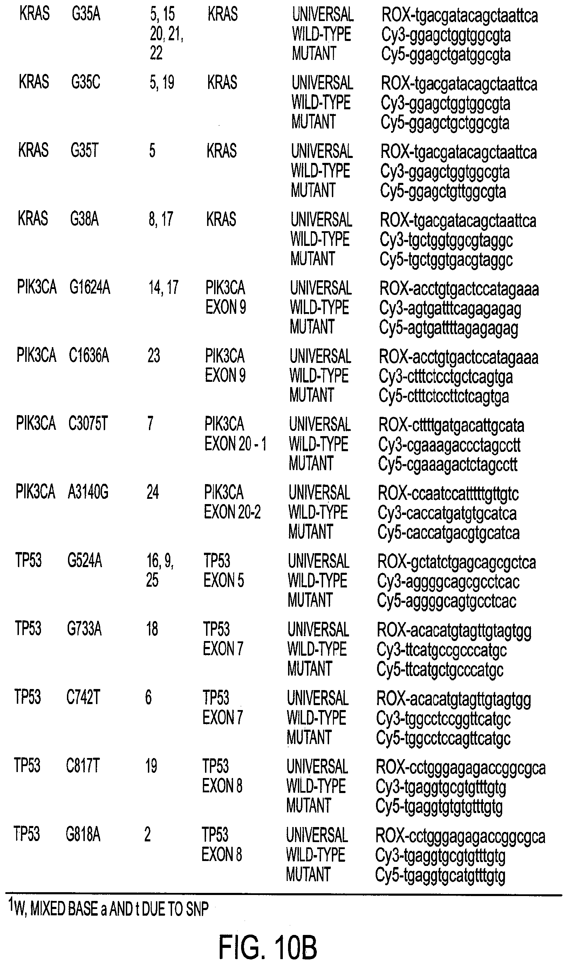

All DNA samples isolated from tumor tissue were analyzed for mutations in 26 regions of APC (19), one region of *KRAS* (1), two regions of PIK3CA (2), and four regions of *TP53* (4) using direct Sanger sequencing. Due to degradation of DNA in FFPE tissue, the amplicon sizes were chosen to be between 74 to 132 bp in length. The first PCR was performed in a 10 µl reaction volume containing 50-100 genome equivalents (GEs) of template DNA (1 GE equals 3.3 pg of human genomic DNA), 0.5 U of Platinum Taq DNA Polymerase (Invitrogen), 1 × PCR buffer (67 mM of Tris-HCl, pH 8.8, 67 mM of MgCl₂, 16.6 mM of (NH₄)₂SO₄, and 10 mM of 2-mercaptoethanol), 2 mM ATP, 6% (v/v) DMSO, 1 mM of each dNTP, and 0.2 µM of each primer. The sequences of the primer sets are listed in Fig. 20. The amplification was carried out under the following conditions: 94°C for 2 min; 3 cycles of 94°C for 15 s, 68°C for 30 s, 70°C for 15 s; 3 cycles of 94°C for 15 s, 65°C for 30 s, 70°C for 15 s, 3 cycles of 94°C for 15 s, 62°C for 30 s, 70°C for 15 s; 40 cycles of 94°C for 15 s, 59°C for 30 s, 70°C for 15 s. One microliter of the first amplification was then added to a second 10-µl PCR reaction mixture of the same makeup as the one described above, except that different primers were used (Fig. 10). The second (nested) PCR reaction was temperature cycled using the following conditions: 2 min at 94°C; 15 cycles of 94°C for 15 s, 58°C for 30 s, 70°C for 15 s. The PCR products were purified using the AMpure system (Agencourt, Beverly, MA) and sequenced from both directions using BigDye Terminator v3.1 (Applied Biosystems). The primers used for sequencing had a 30 bp polyT tag attached to the 5' prime end to improve the sequence quality for the first 30 bases (Tag1 primer: 5'-(dT)₃₀-tcccgcgaaattaatacgac; SEQ ID NO: 1; M13 primer: 5'-(dT)₃₀-gtaaaacgacggccagt; SEQ ID NO: 3). Sequencing reactions were resolved on an automated 96-capillary DNA sequencer (Spectrumedix, State College, PA). Data analysis was performed using Mutation Explorer (SoftGenetics, State College, PA).

### Quantification of total plasma DNA by quantitative real-time PCR

The amount of total DNA isolated from plasma samples was quantified using a modified version of a human LINE-1 quantitative real-time PCR assay¹. Three primer sets were designed to amplify differently sized regions within the most abundant consensus region of the human LINE-1 family (79 bp for: 5'-agggacatggatgaaattgg; SEQ ID NO: 4. 79bp rev: 5'-tgagaatatgcggtgtttgg; SEQ ID NO: 5; 97 bp for: 5'-tggcacatatacaccatggaa; SEQ ID NO: 6, 97 bp rev: 5'-tgagaatgatggtttccaatttc; SEQ ID NO: 7; 127 bp for: 5'-acttggaaccaacccaaatg; SEQ ID NO: 8, 127 bp rev: 5'-tcatccatgtccctacaaagg; SEQ ID NO: 9). PCR was performed in a 25 µl reaction volume consisting of template DNA equal to 2 µl of plasma, 0.5 U of Platinum Taq DNA Polymerase, 1× PCR buffer (see above), 6% (v/v) DMSO, 1 mM of each dNTP, 1:100,000 dilution of SYBR Green I (Invitrogen), and 0.2 µM of each primer. Amplification was carried out in an iCycler (Bio-Rad) using the following cycling conditions: 94°C for 1 min; 2 cycles of 94°C for 10 s, 67°C for 15 s, 70° C for 15 s; 2 cycles of 94° C for 10 s, 64° C for 15 s, 70° C for 15 s, 2 cycles of 94° C for 10 s, 61° C for 15 s, 70° C for 15 s; 35 cycles of 94° C for 10 s, 59° C for 15 s, 70° C for 15 s. Various dilutions of normal human lymphocyte DNA were incorporated in each plate setup to serve as standards. The threshold cycle number was determined using Bio-Rad analysis software with the PCR baseline subtracted. Each quantification was done in duplicate. The total DNA was calculated using the LINE-1 amplicon closest in size to the amplicon being evaluated for mutations (Fig. 11). When the amplicon was equally close to two different LINE-1 amplicons, the average concentation was used. In control experiments with plasma, we found that the number of genome equivalents assessed by the assay of LINE sequences was highly correlated with the number of genome equivalents (GE) *of APC, KRAS, PIK3CA,* or *RAS.* LINE sequence-based assays, rather than these individual genes, were chosen to measure GE because it required a much smaller amount of plasma to measure GE with the former because of its highly repeated nature in the genome.

### BEAMing

Twelve different primer sets were designed for the analysis of 20 mutations (Fig. 11). The DNA purified from 2 ml of plasma was used for each BEAMing assay. An initial amplification with a high fidelity DNA polymerase was performed in eight separate 50 µl PCR reactions each containing template DNA from 250 µl of plasma, 5× Phusion High Fidelity PCR buffer (NEB), 1.5 U of Hotstart Phusion polymerase (NEB), 0.2 µM of each primer, 0.25 mM of each dNTP, and 0.5 mM MgCl₂. Temperature cycling was carried out as described in Fig. 11 Using the primers listed in Fig. 11, a second PCR (nested) was performed by adding 2 µl of the first amplification to a 20-µl PCR reaction of the same makeup as the first one. PCR products were pooled, diluted, and quantified using the PicoGreen dsDNA assay (Invitrogen). The fluorescence intensity was measured using a CytoFluor multiwell plate reader (PE Biosystems) and the DNA quantity was calculated using Lambdaphage DNA reference standards.

Emulsion PCR was performed as described previously². Briefly, a 150 µl PCR mixture was prepared containing 18 pg template DNA, 40 U of Platinum Taq DNA polymerase (Invitrogen), 1 × PCR buffer (see above), 0.2 mM dNTPs, 5 mM MgCl₂, 0.05 µM Tag1 (5'-tcccgcgaaattaatacgac; SEQ ID NO: 1), 8 µM Tag2 (5'-gctggagctctgcagcta; SEQ ID NO: 2) and ∼6x10⁷ magnetic streptavidin beads (MyOne, Invitrogen) coated with Tag1 oligonucleotide (5' dual biotin-T-Spacer18- tcccgcgaaattaatacgac; SEQ ID NO: 1). The 150 µl PCR reaction, 600 µl oil/emulsifier mix (7% ABIL WE09, 20% mineral oil, 73% Tegosoft DEC, Degussa Goldschmidt Chemical, Hopewell, VA), and one 5 mm steel bead (Qiagen) were added to a 96 deep well plate 1.2 ml (Abgene). Emulsions were prepared by shaking the plate in a TissueLyser (Qiagen) for 10 s at 15 Hz and then 7 s at 17 Hz. Emulsions were dispensed into eight PCR wells and temperature cycled at 94°C for 2 min; 3 cycles of 94°C for 10 s, 68°C for 45 s, 70°C for 75 s; 3 cycles of 94°C for 10 s, 65°C for 45 s, 70°C for 75 s, 3 cycles of 94°C for 10 s, 62°C for 45 s, 70°C for 75 s; 50 cycles of 94°C for 10 s, 59°C for 45 s, 70°C for 75 s.

To break the emulsions, 150 µl breaking buffer (10 mM Tris-HCl, pH 7.5, 1% Triton-X 100, 1% SDS, 100 mM NaCl, 1 mM EDTA) was added to each well and mixed with a TissueLyser at 20 Hz for 20 s. Beads were recovered by spinning the suspension at 3,200 g for 2 min and removing the oil phase. The breaking step was repeated twice. All beads from 8 wells were consolidated and washed with 150 µl wash buffer (20 mM Tris-HCl, pH 8.4, 50 mM KCl). The DNA on the beads was denatured for 5 min with 0.1 M NaOH. Finally, beads were washed with 150 µl wash buffer and resuspended in 150 µl of the same buffer.

The mutation status of DNA bound to beads was determined by allele-specific hybridization. Fluorescently labeled probes complementary to the mutant and wild-type DNA sequences were designed for 20 different mutations. The size of the probes ranged from 15 bp to 18 bp depending on the GC content of the target region. All mutant probes were synthesized with a Cy5™ fluorophore on the 5' end and all wild-type probes were coupled to a Cy3™ fluorophore (Integrated DNA Technologies, Coralville, IA, or Biomers, Ulm, Germany). In addition, oligonucleotides that bound to a separate location within the amplicon were used to label every extended PCR product as a positive control. These amplicon specific probes were synthesized with a ROX™ fluorophore attached to their 5' ends. Probe sequences are listed in Fig. 12. Each allele-specific hybridization reaction contained ∼1x10⁷ beads present in 30 µl wash buffer (see above), 66 µl of 1.5× hybridization buffer and heated to 48°C for 5 min to remove unbound probes. After the heating step, beads were again separated magnetically and washed once with 100 pi wash buffer. In the final step, the supernatant was removed and beads resuspended in 200 pi TE buffer for flow cytometric analysis.

A LSR II flow cytometry system (BD Bioscience) equipped with a high throughput autosampler was used for the analysis of each bead population. An average of 5x10⁶ beads were analyzed for each plasma sample. Beads with no extension product were excluded from the analysis. Negative controls, performed using DNA from patients without cancer, were included in each assay. Depending on the mutation being queried, the fraction of beads bound to mutant-specific probes in these negative control samples varied from 0.0061% to 0.00023%. This fraction represented sequence errors introduced by the high fidelity DNA polymerase during the first PCR step, as explained in detail previously³. To be scored as positive in an experimental sample, (i) the fraction of beads bound to mutant fragments had to be higher than the fraction found in the negative control, and (ii) the mean value of mutant DNA fragments per sample plus one standard deviation had to be >1.0. Bead populations generated by BEAMing were analyzed at least twice for each plasma sample.

### References for example 6 only

1. Rago, C. et al. Serial Assessment of Human Tumor Burdens in Mice by the Analysis of Circulating DNA. Cancer Res 67, 9364-9370 (2007).
2. Diehl, F. et al. BEAMing: single-molecule PCR on microparticles in water-in-oil emulsions. Nat Methods 3, 551-9 (2006).
3. Li, M., Diehl, F., Dressman, D., Vogelstein, B. & Kinzler, K.W. BEAMing up for detection and quantification of rare sequence variants. Nat Methods 3, 95-7 (2006).

### Individual Patient Summaries

### Also see Fig.16A-1 to 16E-3

**Patient 1** originally underwent a low anterior resection for rectal carcinoma and was found to have multiple liver metastases with PET/CT scanning. They received post-operative with 5-fluorouracil, oxaliplatin (FOLFOX) and bevacizumab **(Chemotherapy)** for two cycles and repeat imaging revealed a good response. At the time of study entry, the patient underwent right hepatectomy and left lobe wedge resection and cholecystectomy **(Surgery),** followed by chemotherapy with 5-fluorouracil, leucovorin, oxaliplatin and bevacizumab **(Chemotherapy).** Repeat imaging revealed multiple new lung lesions and two new liver lesions. Various other chemotherapy regimens were utilized with continued progression of disease. The patient is currently being considered for a Phase I trial.

**Patient 2** was originally diagnosed with a T3N0M0 colon adenocarcinoma and underwent a left hemicolectomy. At the time of study entry, the patient underwent a right hepatic lobectomy and partial diaphragm resection for metastatic disease **(Surgery).** Repeat imaging studies revealed progressive disease three months following his liver lobectomy and the patient died of disease shortly thereafter.

**Patient 3** was initially found to have metastatic mucinous colon adenocarcinoma, T2N1M1 with a single liver metastasis who underwent a right hemicolectomy with planned liver resection **(Surgery).** However, the patient was found to have diffuse peritoneal implants at the time of surgery, and the liver resection was not performed. Post-operative CT scans revealed evidence of progressive disease with enlarging liver lesion and a new pulmonary nodule. The patient opted to proceed with supportive care only and died of disease approximately one year following his surgery.

**Patient 4** was diagnosed with metastatic colon adenocarcinoma. At study entry, 12 months following the initial surgery, the patient received pre-operative chemotherapy with 5- fluorouracil, oxaliplatin and bevacizumab. The patient then underwent a partial hepatectomy of two liver lesions with radio-frequency ablation of the margins with pathology concurrent with recurrent metastatic adenocarcinoma **(Surgery).** Subsequent CT scans have revealed no evidence of disease recurrence to date.

**Patient 6** originally presented with a T3N1M1 colon adenocarcinoma, and at the time of study entry underwent a right hepatectomy and right lower lobe lung wedge resection **(Surgery).** Follow-up CT scans revealed no evidence of disease and the patient was started on chemotherapy. Eight months later, repeat imaging then revealed a new liver metastasis. The patient then switched to irinotecan, 5-fluorouracil and bevacizumab **(Chemotherapy 1),** but despite four months of therapy still had persistent disease on follow-up CT scans. They were subsequently started on 5-fluorouracil, leucovorin, oxaliplatin and bevacizumab **(Chemotherapy 2)**

**Patient 7** has a prior history of a resected T3N2M1 rectosigmoid adenocarcinoma. At the time of study entry, the patient underwent surgical excision of two recurrent liver lesions, and an additional 4 liver lesions were treated with radiofrequency ablation **(Surgery).** Post-operative imaging revealed no evidence of disease, however, imaging three months later revealed new liver disease and new lung metastases. The patient was started on irinotecan, cetuximab, and bevacizumab **(Chemotherapy).** Despite chemotherapy, on follow-up imaging the patient was noted to have persistent and progressing disease.

**Patient 9** originally presented with a T3N1M0 colon adenocarcinoma followed by adjuvant 5-fluorouracil and leucovorin. At the time of study entry, a solitary liver lesion was noted, and the patient underwent a right hepatectomy, with pathology revealing recurrent adenocarcinoma **(Surgery).** The patient was given post-operative 5-fluorouracil, oxaliplatin and bevacizumab **(Chemotherapy)** and follow up imaging has revealed no evidence of disease recurrence, with evidence of a fully regenerated liver.

**Patient 10** was originally diagnosed with metastatic colorectal adenocarcinoma to the liver and was treated with 5-fluorouracil, oxaliplatin and bevacizumab for four months **(Chemotherapy).** A right hepatectomy and right hemicolectomy was performed **(Surgery 1).** The liver resection was margin positive. Post-operative imaging revealed no evidence of disease. Repeat imaging performed three months later revealed 3 new left liver lesions and the patient subsequently underwent a left liver hepatectomy with radio-frequency ablation to the margins **(Surgery 2).** Post-operative imaging revealed no evidence of disease. At two months follow-up she was found to have boney metastases with a T7 compression fracture for which she underwent external beam radiation.

**Patient 12** was initially diagnosed with metastatic colon adenocarcinoma. At the time of study entry, the patient underwent a repeat partial hepatectomy with radio-frequency ablation **(Surgery)** after achieving some stabilization of disease with 5-fluorouracil, leucovorin, and oxaliplatin **(Chemotherapy).** Post-operative scans revealed no evidence of disease in the liver. However, CT scan of the chest revealed numerous new pulmonary lesions and a follow up PET showed new liver lesions as well. The patient was then referred for a Phase I clinical study.

**Patient 13** had a history of metastatic colon cancer resected from the sigmoid colon, liver and xiphoid process. Approximately 14 months after their original diagnosis, a CT scan revealed a 1cm lesion in the liver, and a follow-up PET scan showed two adjacent foci of disease near the left hepatic lobe. A CT scans performed three months later showed increase in size of the hepatic lesions and a new peritoneal implant. They then underwent resection of the recurrent disease with partial hepatectomy, partial gastrectomy, and partial omentectomy **(Surgery).** Follow-up CT scans performed 1-year following surgery showed hepatic and omental recurrences.

**Patient 14** was found to have colon adenocarcinoma on screening colonoscopy with CT scans showing no evidence of distant metastases. They underwent a sigmoid colectomy **(Surgery)** and pathology revealed a T3N0M0 tumor. No adjuvant chemotherapy was given and they were followed with serial CT scans. The last CT scan showed no evidence of disease.

**Patient 15** had a history of a completely resected T3N1Mx cecal mass and resected umbilical recurrence. Three years after the resection of the primary tumor, a CT scan of the abdomen then revealed a solitary liver metastasis. The patient underwent a right liver hepatectomy **(Surgery).** A follow-up CT scans one month later showed no evidence of disease, but the patient died of disease approximately one year later from recurrent metastatic disease.

**Patient 16** had a rectosigmoid mass on CT after being worked up for bright red blood per rectum, and underwent a sigmoid colectomy **(Surgery).** She was started on 5-fluorouracil, leucovorin and oxaliplatin, which she continued for the next five months **(Chemotherapy).** Follow-up CT scans following completion of therapy has shown no evidence of disease recurrence.

**Patient 17** is a patient with a history of resected colorectal cancer that was found by PET CT to have an isolated liver metastasis in the right lobe. They underwent a right hepatectomy **(Surgery)** and received post-operative chemotherapy with 5-fluorouracil, leucovorin, oxaliplatin and avastin **(Chemotherapy).** She was found to have a recurrence 7 months after surgery.

**Patient 18** was found to have a T3N1Mx adenocarcinoma after undergoing a low anterior resection for a rectal mass. Three years later the patient was noted to have a left hepatic lobe lesion discovered on CT scan imaging. The patient underwent a laparoscopic liver resection **(Surgery).** He received no additional chemotherapy and is currently disease-free.

### References for application excluding examples 5 and 6

1. Wood, L.D. et al. The genomic landscapes of human breast and colorectal cancers. Science 318, 1108-1113 (2007).
2. Nagrath, S. et al. Isolation of rare circulating tumour cells in cancer patients by microchip technology. Nature 450, 1235-1239 (2007).
3. Cristofanilli, M. et al. Circulating tumor cells, disease progression and survival in metastatic breast cancer. N. Engl. J. Med. 351, 781-791 (2004).
4. Sidransky, D. Emerging molecular markers of cancer. Nat. Rev. Cancer 2, 210-219 (2002**).**
5. Goebel, G., Zitt, M., Zitt, M. & Muller, H.M. Circulating nucleic acids in plasma or serum (CNAPS) as prognostic and predictive markers in patients with solid neoplasias. Dis. Markers 21, 105-120 (2005).
6. Fleischhacker, M. & Schmidt, B. Circulating nucleic acids (CNAs) and cancer-a survey. Biochim. Biophys. Acta 1775, 181-232 (2007).
7. Gormally, E., Caboux, E., Vineis, P. & Hainaut, P. Circulating free DNA in plasma or serum as biomarker of carcinogenesis: practical aspects and biological significance. Mutât. Res. 635, 105-117 (2007).
8. Diehl, F. et al. Detection and quantification of mutations in the plasma of patients with colorectal tumors. Proc. Natl. Acad. Sci. USA 102,16368-16373 (2005).
9. Dressman, D., Yan, H., Traverso, G., Kinzler, K.W. & Vogelstein, B. Transforming single DNA molecules into fluorescent magnetic particles for detection and enumeration of genetic variations. Proc. Natl. Acad. Sci. USA 100, 8817-8822 (2003).
10. Goldstein, M.J. & Mitchell, E.P. Carcinoembryonic antigen in the staging and follow-up of patients with colorectal cancer. Cancer Invest. 23, 338-351 (2005).
11. Li, H., Fan, X. & Houghton, J. Tumor microenvironment: the role of the tumor stroma in cancer. J. Cell. Biochem. 101, 805-815 (2007).
12. Hacia, J.G. & Collins, F.S. Mutational analysis using oligonucleotide microarrays. J. Med. Genet. 36, 730-736 (1999).
13. Shendure, J., Mitra, R.D., Varma, C. & Church, G.M. Advanced sequencing technologies: methods and goals. Nat. Rev. Genet. 5, 335-344 (2004).
14. Rago, C. et al. Serial Assessment of human tumor burdens in mice by the analysis of circulating DNA. Cancer Res. 67, 9364-9370 (2007).
15. Diehl, F. et al. BEAMing: single-molecule PCR on microparticles in water-in-oil emulsions. Nat. Methods 3, 551-559 (2006).
16. Li, M., Diehl, F., Dressman, D., Vogelstein, B. & Kinzler, K.W. BEAMing up for detection and quantification of rare sequence variants. Nat. Methods 3, 95-97 (2006)
17. WO 05/10145 A3

## Claims

1. A method to monitor tumor dynamics, comprising the steps of:
testing a tumor sample of a cancer patient and identifying a somatic mutation in a gene selected from the group consisting of *KRAS, APC,* TP53, and *PIK3CA* in the tumor sample; and then
measuring in a test sample of blood or stool of the cancer patient the number of copies of DNA fragments of a gene that have the mutation, wherein the number of copies is an index of the tumor burden in the patient, wherein the method of measuring segregates and assays individual DNA fragments, and wherein the step of measuring is performed on a plurality of test samples obtained from the cancer patient at a plurality of time points to monitor increase, decrease, or stability of tumor burden.

2. The method of claim 1 wherein at least one test sample is obtained before surgery to resect the tumor and at least one test sample is obtained after the surgery.

3. The method of claim 2 further comprising the step of:
testing normal tissue of the patient to determine the absence of the mutation in the gene in the normal tissue.

4. The method of claim 1 further comprising the steps of:
measuring number of copies of DNA fragments of the gene that do not have the mutation in the test sample; and
dividing the number of copies of DNA fragments that have the mutation by the number of copies of DNA fragments of the gene that do not have the mutation in the test sample, to provide a ratio.

5. The method of claim 4 further comprising the step of:
measuring total amount of DNA in the test sample of the cancer patient and normalizing the ratio to the total amount of DNA.

6. The method of claim 1 wherein the cancer patient has had surgery to resect the tumor, and further comprising the steps of:
(1) performing the step of measuring on a test sample obtained from the patient within 2 months of the surgery, and recommending adjuvant therapy if DNA fragments of a gene that have the mutation are detected in the test sample obtained within 2 months of tumor resection;
(2) performing the step of measuring on a test sample obtained from the patient within 1 week of the surgery, and recommending adjuvant therapy if DNA fragments of a gene that have the mutation are detected in the test sample obtained within 1 week of tumor resection;
(3) performing the step of measuring on a test sample obtained from the patient within 1 day of the surgery, and recommending adjuvant therapy if DNA fragments of a gene that have a mutation are detected in the test sample obtained within 1 day of tumor resection;
(4) performing the step of measuring on a test sample obtained from the patient after 2 days of the surgery, and recommending adjuvant therapy if DNA fragments of a gene that have a mutation are detected in the test sample obtained after 2 days post tumor resection; or
(5) performing the step of measuring on a test sample obtained from the patient more than 4 hours of the surgery, and recommending adjuvant therapy if DNA fragments of a gene that have a mutation are detected in the test sample obtained more than 4 hours after of tumor resection.

7. The method of claim 1 wherein the cancer patient has had surgery to resect the tumor, and further comprising the steps of performing the step of measuring on a test sample obtained from the patient after 2 days of the surgery, and predicting tumor recurrence if DNA fragments of a gene that have a mutation are detected after 2 days post tumor resection.

8. The method of claim 1 wherein the tumor sample is tested and the mutation is identified by: hybridization to mutation specific nucleic acid probes.

9. The method of claim 1 wherein the mutation is identified by nucleotide sequencing of tumor DNA of the cancer patient.

10. The method of claim 1 wherein the measuring step employs hybridization to allele-specific nucleic acid probes wherein optionally DNA fragments are thermally denatured prior to hybridization to allele-specific nucleic acid probes, and cooled in the presence of tetramethyl ammonium chloride (TMAC), and wherein preferably the cooling is at least as slow as 0.1°C per second.

11. The method of claim 1 wherein the measuring step employs amplification on a bead in an emulsion, wherein optionally DNA fragments are amplified prior to amplification in an emulsion,

12. The method of claim 1 wherein:
the test sample is stool and the tumor is a colorectal tumor.

13. The method of claim 1 wherein the test sample is blood.

14. The method of claim 1 wherein the tumor is selected from the group consisting of colorectal, breast, lung, kidney, liver, pancreas, stomach, brain, head and neck, lymphatic, ovarian, uterine, bone, and blood.

15. The method of claim 1 wherein the method of measuring is sufficiently sensitive to detect DNA fragments of a gene that have a mutation when said DNA fragments comprise 0.0013% of the total number of said fragments of the gene.

## Patentansprüche

1. Verfahren zum Verfolgen von Tumordynamik, umfassend die Schritte:
Testen einer Tumorprobe eines Krebspatienten und Identifizieren einer somatischen Mutation in einem Gen, das ausgewählt ist aus der Gruppe bestehend aus *KRAS, APC, TP53* und *PIK3CA,* in der Tumorprobe; und anschließend
Messen der Kopienzahl von DNA-Fragmenten eines Gens, die die Mutation haben, in einer Testprobe aus Blut oder Stuhl des Krebspatienten, wobei die Kopienzahl ein Index für die Tumorlast bei dem Patienten ist, wobei das Messverfahren individuelle DNA-Fragmente aufspaltet und analysiert, und wobei der Messschritt an einer Vielzahl von von dem Krebspatienten erhaltenen Testproben an einer Vielzahl von Zeitpunkten durchgeführt wird, um Zunahme, Abnahme oder Stabilität von Tumorlast zu verfolgen.

2. Verfahren nach Anspruch 1, wobei wenigstens eine Testprobe vor Operation zur Resektion des Tumors erhalten wird und wenigstens eine Testprobe nach Operation erhalten wird.

3. Verfahren nach Anspruch 2, weiterhin umfassend den Schritt:
Testen von gesundem Gewebe des Patienten, um das Fehlen der Mutation in dem Gen in dem gesunden Gewebe festzustellen.

4. Verfahren nach Anspruch 1, weiterhin umfassend die Schritte:
Messen der Kopienzahl von DNA-Fragmenten des Gens, die die Mutation nicht haben, in der Testprobe; und
Teilen der Kopienzahl von DNA-Fragmenten in der Testprobe, die die Mutation haben, durch die Kopienzahl von DNA-Fragmenten des Gens, die die Mutation nicht haben, zur Erstellung eines Verhältnisses.

5. Verfahren nach Anspruch 4, weiterhin umfassend den Schritt:
Messen der Gesamtmenge DNA in der Testprobe des Krebspatienten und Normieren des Verhältnisses zur Gesamtmenge DNA.

6. Verfahren nach Anspruch 1, wobei der Krebspatient eine Operation zur Resektion des Tumors hatte, und weiterhin umfassend die Schritte:
(1) Durchführen des Messschritts an einer Testprobe, die innerhalb von 2 Monaten nach der Operation von dem Patienten erhalten wurde, und Empfehlen adjuvanter Therapie, wenn DNA-Fragmente eines Gens, die die Mutation haben, in der innerhalb von 2 Monaten nach Tumorresektion erhaltenen Testprobe nachgewiesen werden;
(2) Durchführen des Messschritts an einer Testprobe, die innerhalb von 1 Woche nach der Operation von dem Patienten erhalten wurde, und Empfehlen adjuvanter Therapie, wenn DNA-Fragmente eines Gens, die die Mutation haben, in der innerhalb von 1 Woche nach Tumorresektion erhaltenen Testprobe nachgewiesen werden;
(3) Durchführen des Messschritts an einer Testprobe, die innerhalb von 1 Tag nach der Operation von dem Patienten erhalten wurde, und Empfehlen adjuvanter Therapie, wenn DNA-Fragmente eines Gens, die eine Mutation haben, in der innerhalb von 1 Tag nach Tumorresektion erhaltenen Testprobe nachgewiesen werden;
(4) Durchführen des Messschritts an einer Testprobe, die nach 2 Tagen nach der Operation von dem Patienten erhalten wurde, und Empfehlen adjuvanter Therapie, wenn DNA-Fragmente eines Gens, die eine Mutation haben, in der nach 2 Tagen nach Tumorresektion erhaltenen Testprobe nachgewiesen werden; oder
(5) Durchführen des Messschritts an einer Testprobe, die mehr als 4 Stunden nach der Operation von dem Patienten erhalten wurde, und Empfehlen adjuvanter Therapie, wenn DNA-Fragmente eines Gens, die eine Mutation haben, in der mehr als 4 Stunden nach Tumorresektion erhaltenen Testprobe nachgewiesen werden.

7. Verfahren nach Anspruch 1, wobei der Krebspatient eine Operation zur Resektion des Tumors hatte, und das weiterhin die Schritte umfasst: Durchführen des Messschritts an einer Testprobe, die nach 2 Tagen nach der Operation von dem Patienten erhalten wurde, und Prognostizieren von Tumorrezidiv, wenn DNA-Fragmente eines Gens, die eine Mutation haben, nach 2 Tagen nach Tumorresektion nachgewiesen werden.

8. Verfahren nach Anspruch 1, wobei die Tumorprobe getestet wird und die Mutation identifiziert wird durch: Hybridisierung an mutationsspezifische Nukleinsäuresonden.

9. Verfahren nach Anspruch 1, wobei die Mutation durch Nukleotidsequenzierung von Tumor-DNA des Krebspatienten identifiziert wird.

10. Verfahren nach Anspruch 1, wobei der Messschritt Hybridisierung an allelspezifische Nukleinsäuresonden verwendet, wobei DNA-Fragmente vor Hybridisierung an allelspezifische Nukleinsäuresonden gegebenenfalls hitzedenaturiert und in Gegenwart von Tetramethylammoniumchlorid (TMAC) abgekühlt werden, und wobei das Abkühlen bevorzugt wenigstens so langsam wie 0,1 °C pro Sekunde ist.

11. Verfahren nach Anspruch 1, wobei der Messschritt Amplifikation an einem Bead in einer Emulsion verwendet, wobei DNA-Fragmente vor Amplifikation in einer Emulsion gegebenenfalls amplifiziert werden.

12. Verfahren nach Anspruch 1, wobei:
die Testprobe Stuhl ist und der Tumor ein Kolorektaltumor ist.

13. Verfahren nach Anspruch 1, wobei die Testprobe Blut ist.

14. Verfahren nach Anspruch 1, wobei der Tumor ausgewählt ist aus der Gruppe bestehend aus Kolorektal-, Brust-, Lungen-, Nieren-, Leber-, Pankreas-, Magen-, Hirn-, Kopf-Hals-, Lymph-, Ovarial-, Uterus-, Knochen- und Bluttomoren.

15. Verfahren nach Anspruch 1, wobei das Messverfahren empfindlich genug ist, um DNA-Fragmente eines Gens, die eine Mutation haben, nachzuweisen, wenn die DNA-Fragmente 0,0013 % der Gesamtzahl der Fragmente des Gens ausmachen,

## Revendications

1. Procédé de surveillance de la dynamique d'une tumeur, comprenant les étapes suivantes :
le test d'un échantillon de tumeur d'un patient souffrant d'un cancer et l'identification d'une mutation somatique dans un gène choisi dans le groupe constitué de *KRAS, APC, TP53,* et *PIK3CA* dans l'échantillon de tumeur ; et ensuite
la mesure dans un échantillon de sang ou de selles à tester du patient souffrant d'un cancer du nombre de copies de fragments d'ADN d'un gène qui présentent la mutation, dans lequel le nombre de copies est un indice de la masse tumorale chez le patient, dans lequel le procédé de mesure sépare et teste des fragments d'ADN individuels, et dans lequel l'étape de mesure est réalisée sur une pluralité d'échantillons à tester prélevés sur le patient souffrant d'un cancer à une pluralité de moments temporels pour surveiller l'augmentation, la diminution ou la stabilité de la masse tumorale.

2. Procédé selon la revendication 1, dans lequel au moins un échantillon à tester est obtenu avant une intervention chirurgicale pour réséquer la tumeur et au moins un échantillon à tester est obtenu après l'intervention chirurgicale.

3. Procédé selon la revendication 2, comprenant en outre l'étape suivante :
le test d'un tissu normal du patient pour déterminer l'absence de la mutation dans le gène du tissu normal.

4. Procédé selon la revendication 1, comprenant en outre l'étape suivante :
la mesure du nombre de copies de fragments d'ADN du gène qui ne présentent pas la mutation dans l'échantillon à tester ; et
la division du nombre de copies de fragments d'ADN qui présentent la mutation par le nombre de copies de fragments d'ADN du gène qui ne présentent pas la mutation dans l'échantillon à tester, pour fournir un rapport.

5. Procédé selon la revendication 4, comprenant en outre l'étape suivante :
la mesure de la quantité totale d'ADN dans l'échantillon à tester du patient souffrant d'un cancer et la normalisation du rapport par rapport à la quantité totale d'ADN.

6. Procédé selon la revendication 1, dans lequel le patient souffrant d'un cancer a subi une intervention chirurgicale pour réséquer la tumeur, et comprenant en outre les étapes suivantes :
(1) la mise en oeuvre de l'étape de mesure sur un échantillon à tester prélevé sur le patient dans les deux 2 mois suivant l'intervention chirurgicale, et la recommandation d'un traitement d'appoint si les fragments d'ADN d'un gène qui présentent la mutation sont détectés dans l'échantillon à tester obtenu dans les 2 mois suivant la résection de la tumeur ;
(2) la mise en oeuvre de l'étape de mesure sur un échantillon à tester prélevé sur le patient dans la semaine suivant l'intervention chirurgicale, et la recommandation d'un traitement d'appoint si les fragments d'ADN d'un gène qui présentent la mutation sont détectés dans l'échantillon à tester obtenu dans la semaine suivant la résection de la tumeur ;
(3) la mise en oeuvre de l'étape de mesure sur un échantillon à tester prélevé sur le patient 1 jour suivant l'intervention chirurgicale, et la recommandation d'un traitement d'appoint si les fragments d'ADN d'un gène qui présentent une mutation sont détectés dans l'échantillon à tester obtenu 1 jour suivant la résection de la tumeur ;
(4) la mise en oeuvre de l'étape de mesure sur un échantillon à tester prélevé sur le patient 2 jours après l'intervention chirurgicale, et la recommandation d'un traitement d'appoint si les fragments d'ADN d'un gène qui présentent une mutation sont détectés dans l'échantillon à tester obtenu après 2 jours post-résection de la tumeur ; ou
(5) la mise en oeuvre de l'étape de mesure sur un échantillon à tester prélevé sur le patient plus de 4 heures après l'intervention chirurgicale, et la recommandation d'un traitement d'appoint si les fragments d'ADN d'un gène qui présentent une mutation sont détectés dans l'échantillon à tester obtenu plus de 4 heures après la résection de la tumeur.

7. Procédé selon la revendication 1, dans lequel le patient souffrant d'un cancer a subi une intervention chirurgicale pour réséquer la tumeur, et comprenant en outre les étapes de mise en oeuvre de l'étape de mesure sur un échantillon à tester prélevé sur le patient 2 jours après l'intervention chirurgicale, et la prédiction de la récurrence de la tumeur si les fragments d'ADN d'un gène qui présentent une mutation sont détectés après 2 jours post-résection de la tumeur.

8. Procédé selon la revendication 1, dans lequel l'échantillon de tumeur est testé et la mutation est identifiée par :
hybridation à des sondes d'acide nucléique spécifiques d'une mutation.

9. Procédé selon la revendication 1, dans lequel la mutation est identifiée par le séquençage des nucléotides de l'ADN de la tumeur du patient souffrant d'un cancer.

10. Procédé selon la revendication 1, dans lequel l'étape de mesure utilise une hybridation à des sondes d'acide nucléique spécifiques d'un allèle, dans lequel facultativement des fragments d'ADN sont dénaturés par voie thermique avant l'hybridation à des sondes d'acide nucléique spécifiques d'un allèle, et refroidis en présence de chlorure de tétraméthyl ammonium (TMAC), et dans lequel de préférence le refroidissement est au moins aussi lent que 0,1 °C par seconde.

11. Procédé selon la revendication 1, dans lequel l'étape de mesure utilise une amplification sur une bille en émulsion, dans lequel facultativement les fragments d'ADN sont amplifiés avant l'amplification en émulsion.

12. Procédé selon la revendication 1, dans lequel :
l'échantillon à tester est un échantillon de selles et la tumeur est une tumeur colorectale.

13. Procédé selon la revendication 1, dans lequel l'échantillon à tester est du sang.

14. Procédé selon la revendication 1, dans lequel la tumeur est choisie dans le groupe constitué d'une tumeur colorectale, du sein, du poumon, du rein, du foie, du pancréas, de l'estomac, du cerveau, de la tête et du cou, lymphatique, des ovaires, de l'utérus, des os, et du sang.

15. Procédé selon la revendication 1, dans lequel le procédé de mesure est suffisamment sensible pour détecter des fragments d'ADN d'un gène qui présentent une mutation quand lesdits fragments d'ADN comprennent 0,0013 % du nombre total desdits fragments du gène.
